# EUROPEAN PATENT APPLICATION

(11) **EP 4 684 804 A1**
(43) Date of publication of application: **28.01.2026**
(21) Application number: 24191313.6
(22) Date of filing: 26.07.2024
(51) Int. Cl.: A61K 47/64, A61P 31/04, A61P 31/12, A61P 31/14

(54) **CONJUGATION PRODUCT COMPRISING AN ANTIGEN BINDING MOIETY AND A MUCIN BINDING MOIETY**

(71) Applicant: Freie Universität Berlin, 14195 Berlin (DE); Forschungsverbund Berlin E.V., 12489 Berlin (DE)
(72) Inventor: LAUSTER, Daniel, 10179 Berlin (DE); BARDUA, Markus, 10439 Berlin (DE); HIMMELSTEIN, Anja, 12249 Berlin (DE); DIEHN, Robyn, 12203 Berlin (DE); TRIMPERT, Jakob, 10115 Berlin (DE); HERRMANN, Andreas, 14467 Potsdam (DE); HACKENBERGER, Christian, 10777 Berlin (DE); FLOREZ, Sebastian, 10407 Berlin (DE)
(74) Representative: Maikowski & Ninnemann Patentanwälte Partnerschaft mbB

(57) **Abstract**

The present invention relates to a conjugation product comprising an antigen binding moiety and a mucin binding moiety covalently bound to the antigen binding moiety. Due to the mucin binding moiety, the antigen binding efficacy of the antigen binding moiety is significantly increased.

## Description

The present invention relates to a conjugation product according to claim 1 and to a method for enhancing an inherent virus-binding efficacy, bacterium-binding efficacy, allergen-binding efficacy, or sperm-cell binding efficacy of an antigen binding moiety according to claim 15.

A significant number of biopharmaceuticals, such as antipathogenic antibodies, fail in clinical trials due to low efficacy. In the respiratory system muco-ciliary clearance can lead to high dilution effects and elimination of biopharmaceuticals. Due to simplicity of the route of administration and dosing, aerosol deposition of biopharmaceuticals is becoming more and more relevant.

WO 2023/187142 A1 describes a peptide comprising a virus binding moiety and a mucin binding moiety covalently bound to the virus binding moiety. This peptide is able to strengthen the mucus by increasing its antimicrobial properties through the incorporation of binding sites for viral pathogens (mucus augmentation). Retained pathogens can then be cleared before reaching the epithelial cell layer, thus preventing infection. The approach described in this international patent application is thus to use modular biomimetic peptides to increase and support the antiviral efficacy of mucus.

Werner Hoffmann (Hoffmann, Werner. "Trefoil factor family (TFF) peptides and their different roles in the mucosal innate immune defense and more: An update." Current Medicinal Chemistry 28.36 (2021): 7387-7399) describes a heterodimer from trefoil factor (TFF3) and IgG-Fc-binding protein (FCGBP).

These prior art approaches aim in augmenting mucus. However, it would also be desirable to increase an inherent efficacy of an antigen binding moiety.

Therefore, it is an object of the present invention to provide substances and methods for increasing an inherent efficacy of an antigen binding moiety.

This object is achieved with a conjugation product having the features of claim 1. Such a conjugation product comprises an antigen binding moiety and a mucin binding moiety which is covalently bound to the antigen binding moiety. The binding between the antigen binding moiety and the mucin binding moiety can be a direct covalent binding or a binding via a linker. Some conjugation products are excluded from the claimed protection. Specifically, the conjugation product in which the antigen binding moiety is IgG-Fc-binding protein (FCGBP) does not form part of the presently claimed subject matter. In addition, if the antigen binding moiety is a peptide having less than 50 amino acids, in particular less than 100 amino acids, in particular less than 150 amino acids, in particular less than 200 amino acids, in particular less than 250 amino acids, the antigen binding moiety must not be a virus binding moiety that comprises or is a peptide being at least 95 % identical to SEQ ID NO. 2, SEQ ID NO. 3, SEQ ID NO. 4, SEQ ID NO. 5, SEQ ID NO. 6, SEQ ID NO. 7, SEQ ID NO. 8, SEQ ID NO. 9, SEQ ID NO. 10, SEQ ID NO. 11, SEQ ID NO. 12, SEQ ID NO. 13, SEQ ID NO. 23, SEQ ID NO. 24, SEQ ID NO. 25, SEQ ID NO. 26, SEQ ID NO. 27, SEQ ID NO. 28, SEQ ID NO. 29, SEQ ID NO. 30, SEQ ID NO. 31, SEQ ID NO. 32, SEQ ID NO. 33, SEQ ID NO. 34, SEQ ID NO. 35, SEQ ID NO. 36, SEQ ID NO. 37, SEQ ID NO. 38, SEQ ID NO. 39, SEQ ID NO. 40, SEQ ID NO. 41, SEQ ID NO. 42, SEQ ID NO. 43, SEQ ID NO. 48, SEQ ID NO. 49, SEQ ID NO. 50, SEQ ID NO. 51, SEQ ID NO. 52, SEQ ID NO. 53, SEQ ID NO. 54, SEQ ID NO. 55, SEQ ID NO. 56, SEQ ID NO. 57, SEQ ID NO. 58, SEQ ID NO. 59, SEQ ID NO. 60, SEQ ID NO. 61, SEQ ID NO. 62, or SEQ ID NO. 63. In an embodiment, such a virus binding moiety is not present in the conjugation product, regardless of the number of amino acids being present in the antigen binding moiety.

The inventors surprisingly found out that by adding a mucin binding moiety to an antigen binding moiety, the functionality of a biopharmaceutical comprising the antigen binding moiety can be tuned towards higher efficacy. While prior art teaches that a derivatization by polyethylene glycol (PEG) (a so-called PEGylation) can be used to increase blood circulation time of antibodies or therapeutic proteins, the efficacy increase of antigen binding moieties described in here presents a totally new and surprising concept of improving the mode of action of biopharmaceuticals.

The addition of the mucus binding moiety to an antigen binding moiety leads to an efficacy increase of therapeutic cytokines, protein vaccines, and many other bioactive substances, which comprise an antigen binding moiety and have protective or immunomodulatory relevance in the mucosal tissue. Further, the claimed conjugation product primarily acts neither on nor in the mucosal host tissue (it only binds to the mucosal host tissue) and thus guarantees as far as possible a low impact on the persons to be treated.

The conjugation product can also be used therapeutically in infected patients, as it will also trap viruses released from infected cells, thereby reducing the viral titer and shedding into the environment. The conjugation product can be combined with established antiviral measures, together helping to reduce the risk of infection and minimizing the risk of disease.

In an embodiment, the mucin binding moiety is chosen from the group consisting of polycarbophil, gelatin, hydroxypropyl methylcellulose, mucoadhesive cellulose-based materials (such as carboxymethylcellulose or ethylcellulose), cationic cellulose derivatives, chitosan, thiolated derivatives of polymers (e.g., thiolated derivatives of chitosan, alginate, or polypeptides), polysulfates (such as hyaluronic acid), polyacrylates, poloxamers, polysaccharides (such as pectins, xyloglucan, lignin, starch, dextran, gum karaya, and derivatives thereof having a sulfation and/or thiolation), sucralfate, maleimide-functionalized polymers for thiol-binding, guar gum, porcine gastric mucins, polyvinyl alcohol, and moieties comprising or being a peptide being at least 95 % %, in particular at least 96 %, in particular at least 97 %, in particular at least 98 %, in particular at least 99 %, in particular 100 %, identical to SEQ ID NO. 1, SEQ ID NO. 14, SEQ ID NO. 18, SEQ ID NO. 19, SEQ ID NO. 20, SEQ ID NO. 21, SEQ ID NO. 22, SEQ ID NO. 64, SEQ ID NO. 65, SEQ ID NO. 66, SEQ ID NO. 67, SEQ ID NO. 68, SEQ ID NO. 69, or SEQ ID NO. 70.

Further details on some of these mucin binding moieties as well as on literature describing these mucin binding moieties can be taken from the following Table 1.

**Table 1: Details on appropriate embodiments of mucin binding moieties.**

| **Name of mucin binding moiety** | **Literature references** |
|---|---|
| Polycarbophil | https://doi.org/10.1111/1.2042-7158.1992.tb03633.x |
| Gelatin | https://doi.org/10.1016/j.ijpharm.2021.121037 |
| Hydroxypropyl methylcellulose (HPMC) | https://doi.org/10.1021/bm5006917 |
| Carboxymethylcellulose, Ethylcellulose, and other mucoashesive cellulose based materials | https://doi.orq/10.1016/i.foodchem.2017.07.134 |
| Cationic cellulose derivatives | https://doi.org/10.1002/admi.202200211 |
| Chitosan | https://doi.org/10.1016/j.reactfunctpolym.2023.105634 |
| Thiolated derivatives of polymers (e.g., thiolated chitosan, alginate, or polypeptides) | https://doi.org/10.1016/i.addr.2005.07.002 |
| Hyaluronic acid, and other polysulfates | https://doi.org/10.1016/j.jmbbm.2023.105908 |
| Polyacrylates | https://doi.org/10.1016/j.ijadhadh.2021.102857 |
| Poloxamer | https://doi.org/10.3390/pharmaceutics13010117 |
| Polysaccharides such as pectines, xyloglucan, lignin, starch, dextrane,gum karaya, and derivatives thereof having a sulfatation or a thiolation | https://doi.org/10.1002/admi.202200211 |
| Sucralfate | https://doi.org/10.1002/admi.202200211 |
| Maleimide-functionalized polymers for thiol-binding | https://doi.org/10.1021/acs.bioconjchem.8b00252 |
| Guar gum and porcine gastric mucins | https://doi.org/10.1016/j.ijbiomac.2023.128390 |
| Polyvinylalcohol | https://doi.org/10.3390/pharmaceutics15061740 |
| Cyclodextrine | https://doi.org/10.3390/polym14153170 |

In an embodiment, the mucin binding moiety is able to bind to at least one of occular, buccal, nasal, respiratory, gastric, intestinal, cervical, vaginal, and urogenital mucus (confer https://doi.org/10.1002/admi.202200211 and further details of these different types of mucus). In an embodiment, the mucin binding moiety is able to bind to at least one of nasal, respiratory, vaginal, and urogenital mucus. In an embodiment, the mucin binding moiety is able to bind at least to nasal mucus. In an embodiment, the mucin binding moiety is able to bind at least to respiratory mucus. In an embodiment, the mucin binding moiety is able to bind at least to vaginal mucus. In an embodiment, the mucin binding moiety is able to bind at least to urogenital mucus.

In an embodiment, the mucin binding moiety comprises or is a peptide or a protein being at least 95 %, in particular at least 96 %, in particular at least 97 %, in particular at least 98 %, in particular at least 99 %, in particular 100 %, identical to SEQ ID NO. 1 (modified trefoil factor 3, TFF3 (C57K)), SEQ ID NO. 14 (native TFF3 (22-80)), SEQ ID NO. 18 (TFF3-C57K(Penty-p6)), SEQ ID NO. 19 (TFF3-p6 rec), SEQ ID NO. 20 (native TFF1), SEQ ID NO. 21 (native TFF2), SEQ ID NO. 22 (jacalin), SEQ ID NO. 64 (native TFF3 (22-80) with GGS sortase acceptor/solubility tag), SEQ ID NO. 65 (native TFF3 (22-80) with GG sortase acceptor/solubility tag), SEQ ID NO. 66 (native TFF3 (22-80) with G sortase acceptor/solubility tag), SEQ ID NO. 67 (TFF3-p6 rec with GGS sortase acceptor/solubility tag), SEQ ID NO. 68 (TFF3-p6 rec with GG sortase acceptor/solubility tag), SEQ ID NO. 69 (TFF3-p6 rec with G sortase acceptor/solubility tag), SEQ ID NO. 70 (TFF3-p6 rec without sortase acceptor/solubility tag), SEQ ID NO. 71 (BanLec (5EXG)), SEQ ID NO. 72 (BanLec (4PIF)), SEQ ID NO. 73 (BanLec (4PIK)), SEQ ID NO. 74 (BanLec (4PIT)), SEQ ID NO. 75 (BanLec (4PIU)), SEQ ID NO. 76 (C-type lectin (1SL4)), SEQ ID NO. 77 (ASGPR-1 (6YAU)), SEQ ID NO. 78 (BDCA-2 (4ZET)), SEQ ID NO. 79 (Bone marrow proteoglycan (2BRS)), SEQ ID NO. 80 (Collectin-K1 (4YLI)), SEQ ID NO. 81 (DC-SIGN (6GHV)), SEQ ID NO. 82 (DC-SIGNR (1K9J)), SEQ ID NO. 83 (DCIR (5B1W)), SEQ ID NO. 84 (Dectin-2 (5VYB)), SEQ ID NO. 85 (E-selectin (1G1T)), SEQ ID NO. 86 (Endo180 (5AO5)), SEQ ID NO. 87 (HIP-PAP (1UV0)), SEQ ID NO. 88 (KLRB1 (5MGR)), SEQ ID NO. 89 (L-selectin (3CFW)), SEQ ID NO. 90 (Langerin (3PF7)), SEQ ID NO. 91 (MBP-C (1KZA)), SEQ ID NO. 92 (Mincle (3WH3)), SEQ ID NO. 93 (P-selectin (1G1Q)), SEQ ID NO. 94 (REG4 (2KV3)), SEQ ID NO. 95 (SP-D (1B08)), SEQ ID NO. 96 (SRCL (2OX8)), SEQ ID NO. 97 (Tetranectin (1HTN)), SEQ ID NO. 98 (I-type lectin (2DF3)), SEQ ID NO. 99 (CD83 (5MIX)), SEQ ID NO. 100 (SIGLEC-2 (5VKJ)), SEQ ID NO. 101 (SIGLEC-3 (6D48)), SEQ ID NO. 102 (SIGLEC-5 (1O7V)), SEQ ID NO. 103 (SIGLEC-7 (1O7V)), SEQ ID NO. 104 (SIGLEC-8 (2N7A)), SEQ ID NO. 105 (SIGLEC-15 (7ZOZ)), SEQ ID NO. 106 (Galectin (1A3K)), SEQ ID NO. 107 (Galectin-1 (1GZW)), SEQ ID NO. 108 (Galectin-2 (5DG1)), SEQ ID NO. 109 (Galectin-3 (1KJL)), SEQ ID NO. 110 (Galectin-4 (4YLZ)), SEQ ID NO. 111 (Galectin-8 (3AP5)), SEQ ID NO. 112 (Galectin-9 (2EAK)), SEQ ID NO. 113 (Galectin-13 (6KJW)), SEQ ID NO. 114 (Ficolin-like lectin (2JHK)), SEQ ID NO. 115 (FIBCD1 (6ZQY)), SEQ ID NO. 116 (H-ficolin (2J64)), SEQ ID NO. 117 (Intelectin 1 (4WMQ)), SEQ ID NO. 118 (L-ficolin (2J3G)), SEQ ID NO. 119 (M-ficolin (2WNP)), SEQ ID NO. 120 (Ricin-like lectin (5AJO)), SEQ ID NO. 121 (CBM13 (3VT1)), SEQ ID NO. 122 (Chi-lectin TIM (4P8V)), SEQ ID NO. 123 (YKL-39 (4AY1)), SEQ ID NO. 124 (Hcgp-39 (1HJV)), SEQ ID NO. 125 (SI-CLP (3BXW)), SEQ ID NO. 126 (Pentraxin (1GYK)), SEQ ID NO. 127 (Calnexin-calreticulin-like lectin (3POW)), SEQ ID NO. 128 (ERGIC-VIP L-type lectin (4GKX)), SEQ ID NO. 129 (P-type lectin (6Z30)), SEQ ID NO. 130 (P-type lectin-like lectin (3AIH)), SEQ ID NO. 131 (Laminin G-like lectin (5IK5)), SEQ ID NO. 132 (Cys-rich man receptor (5XTW)), SEQ ID NO. 133 (F-box (2E33)), SEQ ID NO. 134 (Malectin (2K46)), SEQ ID NO. 135 (Wheat germ agglutinin isolectin 3 (1WGT)), SEQ ID NO. 136 (Wheat germ agglutinin isolectin 1 (4AML)), or SEQ ID NO. 137 (Sambucus nigra lectin (3C9Z)). These lectins and lectin derivatives are particularly appropriate to target a patient's intestinal mucosa. The alphanumeric identifiers given in brackets for SEQ ID. NO. 71 to SEQ ID NO. 137 (e.g., 5EXG) indicate the database access number of the respective protein in the protein data bank and allow access to further details of these proteins. The protein data bank is freely available under the following internet address: https://www.rcsb.org/

TFF3 is a naturally produced protein in the airways (Thim et al. 2002). The naturally occurring TFF3 binds to a glycoepitope (GlcNAc-α-1,4-Gal) only present in secreted mucins (Muc2, Muc5AC, Muc6).

In an embodiment, the mucin binding moiety comprises or is a peptide being at least 95 %, in particular at least 96 %, in particular at least 97 %, in particular at least 98 %, in particular at least 99 %, in particular 100 %, identical to SEQ ID NO. 14 (native TFF3 (22-80)). In an embodiment, this mucin binding moiety is combined with an antibody as antigen binding moiety. In an embodiment, this mucin binding moiety is combined with an antiviral antibody as antigen binding moiety. In an embodiment, this mucin binding moiety is combined with an antibacterial antibody as antigen binding moiety. In an embodiment, this mucin binding moiety is combined with an anti-allergen antibody as antigen binding moiety. In an embodiment, this mucin binding moiety is combined with an anti-sperm cell antibody as antigen binding moiety. A particularly appropriate conjugation product comprises this mucin binding moiety and one of the specific embodiments of an antibody as antigen binding moiety as outlined further below.

In an embodiment, the mucin binding moiety comprises or is a peptide being at least 95 %, in particular at least 96 %, in particular at least 97 %, in particular at least 98 %, in particular at least 99 %, in particular 100 %, identical to SEQ ID NO. 64 (native TFF3 (22-80) with GGS sortase acceptor/solubility tag). In an embodiment, this mucin binding moiety is combined with an antibody as antigen binding moiety. In an embodiment, this mucin binding moiety is combined with an antiviral antibody as antigen binding moiety. In an embodiment, this mucin binding moiety is combined with an antibacterial antibody as antigen binding moiety. In an embodiment, this mucin binding moiety is combined with an anti-allergen antibody as antigen binding moiety. In an embodiment, this mucin binding moiety is combined with an anti-sperm cell antibody as antigen binding moiety. A particularly appropriate conjugation product comprises this mucin binding moiety and one of the specific embodiments of an antibody as antigen binding moiety as outlined further below.

In an embodiment, the mucin binding moiety comprises or is a peptide being at least 95 %, in particular at least 96 %, in particular at least 97 %, in particular at least 98 %, in particular at least 99 %, in particular 100 %, identical to SEQ ID NO. 65 (native TFF3 (22-80) with GG sortase acceptor/solubility tag). In an embodiment, this mucin binding moiety is combined with an antibody as antigen binding moiety. In an embodiment, this mucin binding moiety is combined with an antiviral antibody as antigen binding moiety. In an embodiment, this mucin binding moiety is combined with an antibacterial antibody as antigen binding moiety. In an embodiment, this mucin binding moiety is combined with an anti-allergen antibody as antigen binding moiety. In an embodiment, this mucin binding moiety is combined with an anti-sperm cell antibody as antigen binding moiety. A particularly appropriate conjugation product comprises this mucin binding moiety and one of the specific embodiments of an antibody as antigen binding moiety as outlined further below.

In an embodiment, the mucin binding moiety comprises or is a peptide being at least 95 %, in particular at least 96 %, in particular at least 97 %, in particular at least 98 %, in particular at least 99 %, in particular 100 %, identical to SEQ ID NO. 66 (native TFF3 (22-80) with G sortase acceptor/solubility tag). In an embodiment, this mucin binding moiety is combined with an antibody as antigen binding moiety. In an embodiment, this mucin binding moiety is combined with an antiviral antibody as antigen binding moiety. In an embodiment, this mucin binding moiety is combined with an antibacterial antibody as antigen binding moiety. In an embodiment, this mucin binding moiety is combined with an anti-allergen antibody as antigen binding moiety. In an embodiment, this mucin binding moiety is combined with an anti-sperm cell antibody as antigen binding moiety. A particularly appropriate conjugation product comprises this mucin binding moiety and one of the specific embodiments of an antibody as antigen binding moiety as outlined further below.

In an embodiment, the mucin binding moiety comprises or is a peptide being at least 95 %, in particular at least 96 %, in particular at least 97 %, in particular at least 98 %, in particular at least 99 %, in particular 100 %, identical to SEQ ID NO. 67 (TFF3-p6 rec with GGS sortase acceptor/solubility tag). In an embodiment, this mucin binding moiety is combined with an antibody as antigen binding moiety. In an embodiment, this mucin binding moiety is combined with an antiviral antibody as antigen binding moiety. In an embodiment, this mucin binding moiety is combined with an antibacterial antibody as antigen binding moiety. In an embodiment, this mucin binding moiety is combined with an anti-allergen antibody as antigen binding moiety. In an embodiment, this mucin binding moiety is combined with an anti-sperm cell antibody as antigen binding moiety. A particularly appropriate conjugation product comprises this mucin binding moiety and one of the specific embodiments of an antibody as antigen binding moiety as outlined further below.

In an embodiment, the mucin binding moiety comprises or is a peptide being at least 95 %, in particular at least 96 %, in particular at least 97 %, in particular at least 98 %, in particular at least 99 %, in particular 100 %, identical to SEQ ID NO. 68 (TFF3-p6 rec with GG sortase acceptor/solubility tag). In an embodiment, this mucin binding moiety is combined with an antibody as antigen binding moiety. In an embodiment, this mucin binding moiety is combined with an antiviral antibody as antigen binding moiety. In an embodiment, this mucin binding moiety is combined with an antibacterial antibody as antigen binding moiety. In an embodiment, this mucin binding moiety is combined with an anti-allergen antibody as antigen binding moiety. In an embodiment, this mucin binding moiety is combined with an anti-sperm cell antibody as antigen binding moiety. A particularly appropriate conjugation product comprises this mucin binding moiety and one of the specific embodiments of an antibody as antigen binding moiety as outlined further below.

In an embodiment, the mucin binding moiety comprises or is a peptide being at least 95 %, in particular at least 96 %, in particular at least 97 %, in particular at least 98 %, in particular at least 99 %, in particular 100 %, identical to SEQ ID NO. 69 (TFF3-p6 rec with G sortase acceptor/solubility tag). In an embodiment, this mucin binding moiety is combined with an antibody as antigen binding moiety. In an embodiment, this mucin binding moiety is combined with an antiviral antibody as antigen binding moiety. In an embodiment, this mucin binding moiety is combined with an antibacterial antibody as antigen binding moiety. In an embodiment, this mucin binding moiety is combined with an anti-allergen antibody as antigen binding moiety. In an embodiment, this mucin binding moiety is combined with an anti-sperm cell antibody as antigen binding moiety. A particularly appropriate conjugation product comprises this mucin binding moiety and one of the specific embodiments of an antibody as antigen binding moiety as outlined further below.

In an embodiment, the mucin binding moiety comprises or is a peptide being at least 95 %, in particular at least 96 %, in particular at least 97 %, in particular at least 98 %, in particular at least 99 %, in particular 100 %, identical to SEQ ID NO. 70 (TFF3-p6 rec without sortase acceptor/solubility tag). In an embodiment, this mucin binding moiety is combined with an antibody as antigen binding moiety. In an embodiment, this mucin binding moiety is combined with an antiviral antibody as antigen binding moiety. In an embodiment, this mucin binding moiety is combined with an antibacterial antibody as antigen binding moiety. In an embodiment, this mucin binding moiety is combined with an anti-allergen antibody as antigen binding moiety. In an embodiment, this mucin binding moiety is combined with an anti-sperm cell antibody as antigen binding moiety. A particularly appropriate conjugation product comprises this mucin binding moiety and one of the specific embodiments of an antibody as antigen binding moiety as outlined further below.

In an embodiment, the mucin binding moiety is not an engineered antibody fragment, in particular not an engineered fragment crystallizable region (Fc region), in particular not a polyFc fragment.

In an embodiment, the antigen binding moiety is capable of binding a pathogen (such as a virus, a bacterium, or an allergen) or a sperm cell.

In an embodiment, the antigen binding moiety is a moiety binding a pathogen using the mucus (e.g., mucosal cells) as entry into a host body.

In an embodiment, the antigen binding moiety is a moiety binding a pathogen causing a respiratory disease. In an embodiment, the respiratory disease is chosen from the group consisting of severe acute respiratory syndrome (SARS), Middle East respiratory syndrome (MERS), influenza (e.g., influenza A, or influenza B), infections caused by the respiratory syncytial virus (RSV), infections caused by the human parainfluenza virus (HPIV), and infections caused by the rhinovirus.

In an embodiment, the antigen binding moiety is a moiety binding at least one of SARS-CoV-1, SARS-CoV-2, MERS-CoV, influenza A virus, influenza A/B virus, respiratory syncytial virus, human parainfluenza virus, rhinovirus, orthopoxvirus, herpes simplex virus, hepatitis B virus, hepatitis C virus, varicella zoster virus, canine parvovirus, cytomegalovirus, and human immunodeficiency virus (HIV).

In an embodiment, the antigen binding moiety comprises or is a paratope (such as a nanobody), a foldamer (e.g., a nucleic-acid based foldamer, a peptide-based foldamer, or a polysaccharide-based foldamer), or a polysulfate (e.g., heparan sulfate, a glycosaminoglycan, or a synthetic polysulfate).

In an embodiment, the antigen binding moiety is not a polysulfate if the mucin binding moiety is a polysulfate and vice versa. Thus, in this embodiment, only one of the mucin binding moiety and the antigen binding moiety is a polysulfate.

In an embodiment, the antigen binding moiety comprises a paratope covalently bound to a scaffold protein or a scaffold polymer. The paratope is that part of an antibody that recognizes an epitope of an antigen. It is possible to build up synthetic constructs comprising a paratope of an antibody but lacking the other structural elements of an antibody. Such a case, the paratope can be covalently or coordinatively linked to scaffold protein or scaffold polymer that serves as stabilizing structure while not influencing the epitope-recognizing ability of the paratope. If the mucin binding moiety is bound to such construct of scaffold protein or scaffold protein on the one hand and the paratope on the other hand, the epitope-binding efficacy of the paratope is increased.

In an embodiment, the antigen binding moiety comprises or is a paratope of an antiviral antibody, of an antibacterial antibody, of an anti-allergen antibody, or of an anti-sperm cell antibody.

In an embodiment, the antigen binding moiety is an antiviral antibody, an antibacterial antibody, an anti-allergen antibody, or an anti-sperm cell antibody. The antigen-binding efficacy of such an antibody is enhanced in a particularly appropriate way if the antibody is covalently bound to the mucus binding moiety as defined above.

In an embodiment, the antigen binding moiety is a moiety that is adapted to bind a viral spike protein.

In an embodiment, the antiviral antibody is an antibody targeting at least one of SARS-CoV-1, SARS-CoV-2, MERS-CoV, influenza A virus, influenza A/B virus, respiratory syncytial virus, human parainfluenza virus, rhinovirus, orthopoxvirus, herpes simplex virus, hepatitis B virus, hepatitis C virus, varicella zoster virus, canine parvovirus, cytomegalovirus, and human immunodeficiency virus. In an embodiment, the antiviral antibody is an antibody targeting at least one of SARS-CoV-1, SARS-CoV-2, MERS-CoV, influenza A virus, influenza A/B virus, respiratory syncytial virus, human parainfluenza virus, rhinovirus, i.e., a virus causing a respiratory disease in human or animal being. In an embodiment, the antiviral antibody is an antibody targeting SARS-CoV-1. In an embodiment, the antiviral antibody is an antibody targeting SARS-CoV-2. In an embodiment, the antiviral antibody is an antibody targeting MERS-CoV. In an embodiment, the antiviral antibody is an antibody targeting influenza A/B virus. In an embodiment, the antiviral antibody is an antibody targeting respiratory syncytial virus. In an embodiment, the antiviral antibody is an antibody targeting human parainfluenza virus. In an embodiment, the antiviral antibody is an antibody targeting rhinovirus.

In an embodiment, the antiviral antibody is chosen from the group consisting of REGN10989, REGN10986, REGN10934, REGN10964, REGN10954, REGN10984, adintrevimab, amubarvimab, bamlanivimab, bebtelovimab, beludavimab, canrivitug, casirivimab, cilgavimab, crexavibart, ensovibep, enuzovimab, etesevimab, gorivitug, imdevimab, lomtegovimab, masavibart, nepuvibart, nisfevitug, ogalvibart, pemivibart, plutavimab, regdanvimab, rimteravimab, romlusevimab, simaravibart, sipavibart, sotrovimab, timcevibart, tixagevimab, upanovimab, CR3014, m396, REGN3051, REGN3048, m336, LCA60, 4C2, 2E6, 3B11-N, MERS-4, MERS-27, firivumab, gedivumab, navivumab, CR9114, VIS410, FI6, MEDI8852, VIR-2482, MHAA4549A, 2G02, FNI9, FI6v3, clesrovimab, felvizumab, gontivimab, motavizumab, nirsevimab, palivizumab, suptavumab, HNK20, RSV19, PI3-E12, PI3-C9, PI3-A3, PI3-B5, PI3-A10, PI3-A12, PIA174, anti-14VP1, anti-89VP1, MV33, EV42, Fd138-80, Fd79, lenvervimab, AB65, AB68, TI-57, anivovetmab, fiztasovimab, regavirumab, sevirumab, alvircept sudotox, elipovimab, lafuvitug, teropavimab, and zinlirvimab.

Further details on these antibodies as well as on literature describing these antibodies can be taken from the following Table 2.

**Table 2: Details on appropriate embodiments of antiviral antibodies.**

| **Target** | **INN** | **Common name** | **Literature references** |
|---|---|---|---|
| SARS-CoV-2 | | REGN10989 | https://doi.org/10.1126/science.abd0831 |
| | | REGN10986 | |
| | | REGN10934 | |
| | | REGN10964 | |
| | | REGN10954 | |
| | | REGN10984 | |
| SARS-CoV-2 | adintrevimab | ADG-20 | https://doi.org/10.1128/aac.01353-22 |
| | | ADG-2 | |
| SARS-CoV-2 | amubarvimab | BRII-196 | https://doi.org/10.1007/s40265-022-01759-3 |
| | | BRII196 | |
| | | BRII 196 | |
| SARS-CoV-2 | bamlanivimab | LY3819253 | https://doi.org/10.1038/s41467-022-32551-2 |
| | | LY-3819253 | |
| | | LY-CoV555 | |
| SARS-CoV-2 | bebtelovimab | LY-CoV-1404 | https://doi.orq/10.1016/S1473-3099(22)00592-8 |
| | | LY-3853113 | |
| | | LY 3853113 | |
| SARS-CoV-2 | beludavimab | VIR-7832 | https://doi.org/10.1101/2021.03.09.434607 |
| | | GSK4182137 | |
| | | WBP2271 | |
| SARS-CoV-2 | canrivitug | | |
| SARS-CoV-2 | casirivimab | REGN10933 | https://doi.org/10.1126/science.abd0831 |
| | | REGN-10933 | |
| SARS-CoV-2 | cilgavimab | AZD1061 | https://doi.org/10.1056/NEJMoa2116620 |
| | | COV2-2130 | |
| | | 2130 | |
| | | AZD7442 | |
| SARS-CoV-2 | crexavibart | C-144-LS | |
| | | BMS-986413 | |
| | | BMS 986413 | |
| | | BMS986413 | |
| SARS-CoV-2 | ensovibep | MP0420 | https://doi.org/10.1101/2021.02.03.429164 |
| | | MP-0420 | |
| SARS-CoV-2 | enuzovimab | HFB30132A | |
| | | HFB-30132A | |
| | | ABL901 | |
| | | ABL-901 | |
| SARS-CoV-2 | etesevimab | LY3832479 | https://doi.org/10.1056/NEJMoa2102685 |
| | | CB-6 | |
| | | JS-016 | |
| SARS-CoV-2 | gorivitug | REGN-15160 | |
| | | REGN15160 | |
| SARS-CoV-2 | imdevimab | REGN10987 | https://doi.org/10.1126/science.abd0831 |
| | | REGN-10987 | |
| SARS-CoV-2 | lomtegovimab | BI 767551 | https://doi.org/10.3390/v13081498 |
| | | BI-767551 | |
| | | EX-14870 | |
| | | EX 14870 | |
| | | DZIF-10c | |
| SARS-CoV-2 | masavibart | ZRC-3308A7 | |
| | | ZRC-3308 | |
| SARS-CoV-2 | nepuvibart | ZRC-3308 | |
| | | ZRC- | |
| | | 3308B10 | |
| SARS-CoV-2 | nisfevitug | | |
| SARS-CoV-2 | ogalvibart | C-135-LS | |
| | | BMS-986414 | |
| | | BMS 986414 | |
| | | BMS986414 | |
| SARS-CoV-2 | pemivibart | | |
| SARS-CoV-2 | plutavimab | STI-2020 | |
| | | STI 2020 | |
| | | STI2020 | |
| SARS-CoV-2 | regdanvimab | CT-P59 | |
| | | CT-P-59 | |
| SARS-CoV-2 | rimteravimab | XVR011 | |
| | | VHH-72-Fc | |
| SARS-CoV-2 | romlusevimab | BRII-198 | https://doi.org/10.1007/s40265-022-01759-3 |
| | | BRII198 | |
| | | BRII 198 | |
| SARS-CoV-2 | simaravibart | MAD- | |
| | | 0004J08 | |
| | | MAD0004J08 | |
| SARS-CoV-2 | sipavibart | | |
| SARS-CoV-2 | sotrovimab | VIR-7831 | https://doi.org/10.1001/jama.2022.2832 |
| SARS-CoV-2 | timcevibart | | |
| SARS-CoV-2 | tixagevimab | AZD8895 | https://doi.org/10.1056/NEJMoa2116620 |
| | | AZD7442 | |
| SARS-CoV-2 | upanovimab | SCTA01 | |
| | | SCTA-01 | |
| | | SCTA 01 | |
| SARS-CoV-1 | | CR3014 | https://doi.org/10.1371/journal.pmed.0030237 |
| SARS-CoV-1 | | m396 | https://doi.org/10.1073/pnas.0701000104 |
| | | | |
| MERS-CoV | | REGN3051 | PMID: 33507266 |
| MERS-CoV | | REGN3048 | PMID: 33507266 |
| MERS-CoV | | m336 | https://doi.org/10.1038/ncomms9223 |
| MERS-CoV | | LCA60 | https://doi.org/10.1016/j.antiviral.2019.01.016 |
| MERS-CoV | | 4C2 | PMID: 26391698 |
| MERS-CoV | | 2E6 | PMID: 26391698 |
| MERS-CoV | | 3B11-N | PMID: 26828465 |
| MERS-CoV | | MERS-4 | https://doi.org/10.1126/scitranslmed.3008140 |
| MERS-CoV | | MERS-27 | https://doi.org/10.1126/scitranslmed.3008140 |
| | | | |
| Influenza A | firivumab | CT-P22 | |
| Influenza A | gedivumab | MHAA4549A | |
| | | RO6876802 | |
| | | RG7745 | |
| Influenza A | navivumab | CT-P23 | |
| Influenza A/B | | CR9114 | https://doi.org/10.3389/fviro.2022.1049134 |
| Influenza A | | VIS410 | PMID: 30638863 |
| Influenza A | | FI6 | https://doi.org/10.1126/science.1205669 |
| Influenza A | | MEDI8852 | https://doi.org/10.1016/j.cell.2016.05.073 |
| Influenza A | | VIR-2482 | PMID: 38376227 |
| Influenza A | | MHAA4549A | https://doi.org/10.1093/ofid/ofab630 |
| Influenza A | | 2G02 | https://doi.org/10.1128/JVI.00949-18 |
| Influenza A | | FNI9 | PMID: 37258672 |
| Influenza A | | FI6v3 | https://doi.org/10.1126/science.1205669 |
| | | | |
| RSV | clesrovimab | MK-1654 | https://doi.org/10.1038/d41586-023-02957-z |
| | | MK1654 | |
| | | MK 1654 | |
| RSV | felvizumab | SB 209763 | |
| RSV | gontivimab | ALX-0171 | |
| | | VR-465 | |
| RSV | motavizumab | MEDI-524 | |
| RSV | nirsevimab | MEDI8897 | |
| | | MEDI-8897 | |
| | | nirsevimab-alip | |
| RSV | palivizumab | MEDI-493 | |
| RSV | suptavumab | REGN2222 | |
| | | SAR438584 | |
| | | REGN 2222 | |
| | | REGN-2222 | |
| | | SAR-438584 | |
| | | H1H3592P3 | |
| RSV | | HNK20 | |
| RSV | | RSV19 | |
| | | | |
| HPIV | | PI3-E12 | https://doi.org/10.1080/19420862.2021.1912884 |
| HPIV | | PI3-C9 | https://doi.org/10.1080/19420862.2021.1912884 |
| HPIV | | PI3-A3 | https://doi.org/10.1080/19420862.2021.1912884 |
| HPIV | | PI3-B5 | https://doi.org/10.1080/19420862.2021.1912884 |
| HPIV | | PI3-A10 | https://doi.org/10.1080/19420862.2021.1912884 |
| HPIV | | PI3-A12 | https://doi.org/10.1080/19420862.2021.1912884 |
| HPIV | | PIA174 | https://doi.org/10.1080/19420862.2021.1912884 |
| | | | |
| Rhinovirus | | anti-14VP1 | https://doi.orcl/10.1183/09031936.00149109 |
| Rhinovirus | | anti-89VP1 | https://doi.org/10.1183/09031936.00149109 |
| | | | |
| Orthopoxvirus | | MV33 | https://doi.org/10.1038/s41467-024-47328-y |
| Orthopoxvirus | | EV42 | https://doi.org/10.1038/s41467-024-47328-y |
| | | | |
| Herpes simplex virus (HSV-1) | | Fd138-80 | PMID: 1849279 |
| Herpes simplex virus (HSV-1) | | Fd79 | PMID: 1849279 |
| | | | |
| Hepatitis B (HBV) | lenvervimab | GC1102 | https://doi.org/10.1016/i.cgh.2019.09.038 |
| | | | |
| Hepatitis C (HCV) | | AB65 | PMID: 15600247 |
| | | AbXTL65 | |
| | | HepeX-C | |
| | | | |
| Hepatitis C (HCV) | | AB68 | PMID: 15600247 |
| | | AbXTL68 | |
| | | HepeX-C | |
| | | | |
| Varicella zoster virus (human herpesvirus 3) | | TI-57 | https://doi.org/10.1128/JVI.02097-08 |
| | | TI57 | |
| | | V3 | |
| | | | |
| Canine parvovirus | anivovetmab | KIND-030 | https://drugs.ncats.io/substance/GZ8Z492VVR |
| | | | |
| Cytomegalovirus (human herpesvirus 5) | fiztasovimab | NPC-21 | PMID: 34989174 |
| **Cytomegalovirus** (human herpesvirus 5) | regavirumab | **C23** | PMID: 7945529 |
| | | MCA C23 | |
| | | TI-23 | |
| Cytomegalovirus (human herpesvirus 5) | sevirumab | EV2 7 SDZ | PMID: 9207350 |
| | | MSL 109 | |
| | | MSL 109 | |
| | | | |
| Human immunodeficiency virus (HIV) | alvircept sudotox | sCD4-PE40 | https://drugs.ncats.io/substance/Q67R7N520V |
| | | CD4(178) | |
| | | PE40 | |
| Human immunodeficiency virus (HIV) | elipovimab | GS-HIV | PMID: 32604408 |
| Human immunodeficiency virus (HIV) | lafuvitug | | https://drugs.neats.io/substance/IS3OH21U4J |
| Human immunodeficiency virus (HIV) | teropavimab | KD-247 | PMID: 16699037 |
| Human immunodeficiency virus (HIV) | zinlirvimab | GS-5423 | PMID: 37265259 |
| | | 3BNC117-LS | |
| | | GS-2872 | |
| | | GS 2872 | |
| | | GS2872 | |

In an embodiment, in which the antigen binding moiety comprises 50 or more amino acids, in particular 100 or more amino acids, in particular 150 or more amino acids, in particular 200 or more amino acids, in particular 250 or more amino acids, the antigen binding moiety is a virus binding moiety, in particular an antiviral antibody, comprising a peptide being at least 95 %, in particular at least 96 %, in particular at least 97 %, in particular at least 98 %, in particular at least 99 %, in particular 100 %, identical to SEQ ID NO. 2, SEQ ID NO. 3, SEQ ID NO. 23, SEQ ID NO. 24, SEQ ID NO. 25, SEQ ID NO. 26, SEQ ID NO. 27, SEQ ID NO. 28, SEQ ID NO. 29, SEQ ID NO. 30, SEQ ID NO. 31, SEQ ID NO. 32, SEQ ID NO. 33, SEQ ID NO. 34, SEQ ID NO. 35, SEQ ID NO. 36, SEQ ID NO. 37, SEQ ID NO. 38, SEQ ID NO. 39, SEQ ID NO. 40, SEQ ID NO. 41, SEQ ID NO. 48, SEQ ID NO. 49, SEQ ID NO. 50, SEQ ID NO. 51, SEQ ID NO. 52, SEQ ID NO. 53, SEQ ID NO. 54, SEQ ID NO. 55, SEQ ID NO. 56, SEQ ID NO. 57, SEQ ID NO. 58, SEQ ID NO. 59, SEQ ID NO. 60, SEQ ID NO. 61, SEQ ID NO. 62, or SEQ ID NO. 63.

SEQ ID NO. 2, 3 and 23 to 41 relate to linear virus binding moieties that are particularly appropriate for binding the severe acute respiratory syndrome coronavirus 2 (SARS-CoV-2).

SEQ ID NO. 48 describes a peptide particularly appropriate for binding the influenza A virus (in particular strains H1, H3, H7). This peptide targets the hemagglutinin (HA protein) of the influenza A virus and is described by Memczak et al. and Miriam Hoffmann et al.

SEQ ID NO. 49 and 50 describe peptides particularly appropriate for binding the influenza A virus (in particular strain H5N1 clade 2.3.4, bird strain). These peptides target the hemagglutinin (HA protein) of the influenza A virus and are derived from sequences of antibody 65C6 described by Zuo et al.

SEQ ID NO. 51 and 52 describe peptides particularly appropriate for binding the influenza A virus (in particular strain H5N1 clade 2.3.2.1, bird strain). These peptides target specific sequences of the hemagglutinin (HA protein) found in general in HA protein of variants of this strain, for example in the sequences of the HA protein described by Bui et al.

SEQ ID NO. 53 and 54 describe peptides particularly appropriate for binding the influenza B virus. These peptides target the neuraminidase of the influenza B virus and are described by Madsen et al.

SEQ ID NO. 55 and 56 describe peptides particularly appropriate for binding rhinoviruses (RVs) and other enteroviruses (EV). These peptides are derived from anti-RV antibodies that are described by Hrebik et al., Khan et al., and Smith et al.

SEQ ID NO. 57 describes a peptide particularly appropriate for binding the human parainfluenza virus (HPIV) (in particular strains HIPV1 and HIPV3). This peptide targets the fusion protein (F protein) of HPIV and is derived from anti-HPIV antibodies described by Cabán et al.

SEQ ID NO. 58, 59, and 60 describe peptides particularly appropriate for binding the metapneuvirus (MPV) and the respiratory syncytial virus (RSV) (in particular subtype A and/or subtype B). These peptides target the fusion protein (F protein) of MPV and of RSV. They are derived from anti-HPIV antibodies and are described by Cabán et al.

SEQ ID NO. 61, 62, and 63 describe peptides particularly appropriate for binding RSV (in particular subtype A and/or subtype B). These peptides also target the fusion protein (F protein) of RSV and are described by Issmail et al, Zhu et al., and McLellan et al.

In an embodiment, the antibacterial antibody is an antibody targeting at least one of Pseudomonas aeruginosa, Staphylococcus aureus, Vibrio cholerae, enterotoxigenic Escherichia coli (EHEC), Shiga toxin-producing E. coli (STEC), and Neisseria gonorrhoeae.

Panobacumab (an IgM antibody), aerucin (an anti-IgG antibody), COT-143 (an anti-IgG antibody), and DSTA4637S (an anti-IgG antibody-antibiotic conjugate) are particularly appropriate examples for such antibacterial antibodies.

Further details on these antibodies as well as on literature describing these antibodies can be taken from the following Table 3.

**Table 3: Details on appropriate embodiments of antibacterial antibodies.**

| **Target** | **INN** | **Common name** | **Literature references** |
|---|---|---|---|
| Pseudomonas aeruginosa | Panobacumab | | https://doi.org/10.1007/s10096-014-2156-1 |
| Pseudomonas aeruginosa | | Aerucin | https://doi.org/10.3390/microorganisms11040916 |
| Pseudomonas aeruginosa | | COT-143 | |
| Staphylococcus aureus | | DSTA4637S | https://doi.org/10.1093/ofid/ofaa439.477 |
| Vibrio cholerae | | IgA | https://doi.org/10.1128/mbio.02847-20 |
| | | IgG | |
| | | IGM | |
| enterotoxigenic Escherichia coli (EHEC) | | Anti-adhesin factors (CS1-CS6, CD21, EptA, CFA) | https://doi.org/10.1371%2Fjournal.pone.0216076 |
| Shiga toxin-producing E. coli (STEC) | | Anti-Stx1 | https://doi.org/10.3389/fcimb.2022.825856 |
| | | Anti-Stx2 | |
| Neisseria gonorrhoeae | | Anti-LOS | https://doi.org/10.1038/s41598-024-55606-4 https://doi.org/10.13028/M2CD5K |

In an embodiment, the anti-allergen antibody is an antibody targeting at least one of house dust mite (HDM), peanut, cat hairs, and pollen, and/or is chosen from the group consisting of omalizumab (an anti-IgE antibody), mepolizumab (an anti-IL5 antibody), reslizumab (an anti-IL5 antibody), benralizumab (an anti-IL5 receptor alpha antibody), dupilumab (an anti-IL4 receptor alpha antibody), house dust mite Fab (antibody serum of patients contacted with house dust mite (Dermatophagoides pteronyssinus)), house dust mite IgE, peanut-specific IgG, peanut-Fab, cat-Fel d 1, cat-Fel d 2, Bet v 1-IgE (directed against birch pollen allergens), and Phl p 5-Fab (directed against grass pollen allergens).

Further details on these antibodies as well as on literature describing these antibodies can be taken from the following Table 4.

**Table 4: Details on appropriate embodiments of anti-allergen antibodies.**

| **Target/disease to be treated** | **INN** | **Common name** | **Literature references** |
|---|---|---|---|
| Asthma | Omalizumab | | https://doi.org/10.1056/NEJMoa2312382 |
| Asthma | Mepolizumab | | https://doi.org/10.1056/NEJMoa1403290 |
| Asthma | Reslizumab | | https://doi.org/10.1016/S2213-2600(15)00042-9 |
| Eosinophilic granulomatosis with polyangiitis | Benralizumab | | https://doi.org/10.101 6/S2665-9913(23)00243-6 |
| Eosinophilic granulomatosis with polyangiitis | Dupilumab | | https://doi.org/10.1007/s13555-022-00778-y |
| House dust mite | | HDM-Fab | https://doi.org/10.1016/j.anai.2022.03.031 |
| House dust mite | | HDM-IgE | https://doi.org/10.1159/000236176 |
| Peanut | | Peanut-Specific IgG | https://doi.org/10.1016/j.jaci.2014.05.042 |
| Peanut | | Peanut-Fab | |
| Cat | | Cat-Fel d 1 | https://doi.org/10.1155/2021/5545173 |
| Cat | | Cat-Fel d 2 | https://doi.org/10.1155/2021/5545173 |
| Pollen | | Bet v 1-IgE | https://doi.org/10.1111/all.15865 |
| Pollen | | Phl p 5-Fab | https://doi.org/10.4049/jimmunol.165.7.3849 |

In an embodiment, the anti-sperm cell antibody is chosen from the group consisting of anti-sperm IgA, anti-sperm IgG, and anti-sperm IgM.

Further details on these antibodies as well as on literature describing these antibodies can be taken from the following Table 5.

**Table 5: Details on appropriate embodiments of anti-sperm cell antibodies.**

| **Target** | **INN** | **Common name** | **Literature references** |
|---|---|---|---|
| Sperm Cells | | Anti-sperm IgA | https://doi.org/10.1530/REP-21-0123 |
| Sperm Cells | | Anti-sperm IgG | https://doi.org/10.1530/REP-21-0123 |
| Sperm Cells | | Anti-sperm IgM | https://doi.org/10.1530/REP-21-0123 |

An appropriate linker for establishing a covalent binding between the mucin binding moiety and the antigen binding moiety is a polyethylene glycol (PEG) linker or an amino acid linker comprising 1 to 20 amino acids, in particular 2 to 19 amino acids, in particular 3 to 18 amino acids, in particular 4 to 17 amino acids, in particular 5 to 16 amino acids, in particular 6 to 15 amino acids, in particular 7 to 14 amino acids, in particular 8 to 13 amino acids, in particular 9 to 12 amino acids, in particular 10 to 11 amino acids. A dipeptide, such as a GT dipeptide, is a particularly appropriate linker. A linker comprising or consisting of an amino acid sequence being at least 95 % %, in particular at least 96 %, in particular at least 97 %, in particular at least 98 %, in particular at least 99 %, in particular 100 % identical to SEQ ID NO. 44, SEQ ID NO. 45, SEQ ID NO. 46, or SEQ ID NO. 47 it is a particularly appropriate linker, too. SEQ ID NO. 44 is an example for a flexible linker. SEQ ID NO. 45 is an example for a rigid linker. SEQ ID NO. 46 represents a linking peptide resulting from a chemo-enzymatic coupling reaction via Sortase A. SEQ ID NO. 47 represents a linking peptide resulting from a chemo-enzymatic coupling reaction via transglutaminase. The antigen binding moiety or the mucin binding moiety peptide is coupled to the glutamyl residue (Q7) via a γ-glutamyl-ε-lysyl isopeptide bond. In an embodiment, the mucin binding moiety comprises a glycotope binding domain and binds to an N-acetylglucosamine-alpha-1,4-galactose (GlcNAc-α-1,4-Gal) disaccharide with an affinity of at least 150 µM, in particular of at least 100 µM, in particular of at least 75 µM, in particular of at least 50 µM, in particular of at least 25 µM, in particular with an affinity lying in a range of from 25 µM to 150 µM, in particular from 50 µM to 125 µM, in particular from 75 µM to 100 µM. The GlcNAc-α-1,4-Gal disaccharide is a glycotope that is specifically present in mucins such as mucin 2 (Muc2), mucin 5AC (Muc5AC), and mucin 6 (Muc6). To give an example, natural trefoil factor 3 (TFF3) has a binding affinity towards the GlcNAc-α-1,4-Gal disaccharide of 56 µM (Järvå et al 2020).

In an embodiment, the conjugation product comprises at least two antigen binding moieties. In this context, the mucin binding moiety is directly or via a linker covalently bound to at least one of the at least two antigen binding moieties. The mucin binding moiety may also be bound to more than one antigen binding moieties. For this purpose, a bifurcated linker is, in an embodiment, particularly helpful. Such a bifurcated linker may be used to couple a single mucin binding moiety to a plurality of antigen binding moieties that are, after coupling, arranged more or less equally distant from the mucin binding moiety.

In an embodiment, the conjugation product comprises more than one mucin binding moieties per antigen binding moiety. By increasing the number of mucin binding moieties in the produced conjugation product, i.e., by increasing the ratio between mucin binding moieties to antigen binding moieties in the conjugation product, the efficacy of the antigenic moiety against antigens, in particular the anti-pathogenic efficacy of antibodies used as antigenic moiety is increased. in an embodiment, the ratio between mucin binding moieties to antigen binding moieties lies in a range from 1.5 to 10, in particular from 2 to 9, in particular from 2.5 to 8, in particular from 3 to 7, in particular from 3.5 to 6.5, in particular from 4 to 6, in particular from 4.5 to 5.

In an aspect, the present invention relates to the use of the conjugation product according to the preceding explanations as medicament.

In an aspect, the present invention relates to the medical use of the conjugation product according to the preceding explanations in preventing or treating a viral infection, a bacterial infection, or an allergic reaction.

In an embodiment, the viral infection to be treated or prevented is an infection with SARS-CoV-1. In an embodiment, the viral infection to be treated or prevented is an infection with SARS-CoV-2. In an embodiment, the viral infection to be treated or prevented is an infection with MERS-CoV. In an embodiment, the viral infection to be treated or prevented is an infection with influenza A virus. In an embodiment, the viral infection to be treated or prevented is an infection with influenza B virus. In an embodiment, the viral infection to be treated or prevented is an infection with influenza A/B virus. In an embodiment, the viral infection to be treated or prevented is an infection with respiratory syncytial virus. In an embodiment, the viral infection to be treated or prevented is an infection with human parainfluenza virus. In an embodiment, the viral infection to be treated or prevented is an infection with rhinovirus.

In an aspect, the present invention relates to the (medical) use of the conjugation product according to the preceding explanations in preventing the fertilization of an oocyte by a sperm cell, i.e. to the use of the conjugation product as contraceptive.

In an aspect, the present invention relates to a medical method for treating or preventing a viral infection, a bacterial infection, or an allergic reaction in a patient in need of such treatment. The term "patient" as used herein relates to a human or animal patient. Mammals are particularly appropriate animal patients. The method comprises the step of administering the conjugation product according to the preceding explanations to the patient (e.g., in form of a medicament comprising this conjugation product). This administration is done, in an embodiment, by providing the conjugation product to the nose or to the mouth of the patient. This can be particularly easy achieved by providing the conjugation product in a dosage form of a spray. Then, the conjugation product can be administered, e.g., as an aerosol like a nasal spray or an inhalation spray. Upon administration, it will then contact the patient's mucosa and will allow a binding of the conjugation product to the mucosa via the mucin binding moiety. Afterwards, the conjugation product will stay in its bound form at the patient's mucosa and will be able to "filter" viruses, bacteria, and/or allergens out of the air inhaled by the patient due to its antigen binding moiety.

In an aspect, the present invention relates to a medical method for contraception in a (female) animal or human person in need thereof. This method comprises the step of administering the conjugation product according to the preceding explanations to the person (e.g., in form of a medicament comprising this conjugation product), in particular to the vaginal or urogenital mucus, e.g., in form of a suppository. Upon administration, the conjugation product will then contact the person's mucosa and will allow a binding of the conjugation product to the mucosa via the mucin binding moiety. Afterwards, the conjugation product will stay in its bound form at the patient's mucosa and will be able, due to its antigen binding moiety, to "filter" out sperm cells passing by.

In an aspect, the present invention relates to a medicament that comprises a conjugation product according to the preceding explanations as pharmaceutically active ingredient. This medicament can comprise other pharmaceutically active ingredients. In an embodiment, the conjugation product is the only pharmaceutically active ingredient.

In an embodiment, the medicament is formulated such that it can be administered as an aerosol, such as a nasal spray or as an inhalation spray. Then, the medicament (and therewith the conjugation product as pharmaceutically active ingredient) directly contacts the mucosa in the nasopharyngeal zone upon its administration. Due to the efficacy-enhancing effect of the conjugation product with respect to the free antigen binding moiety, the patient's mucus is better prepared to bind and subsequently eliminate viruses, bacteria, and other antigens with which the patient is confronted (either by inhaling them from the environment or by exhaling them from infected cells of the patient's body).

In an aspect, the present invention relates to a method for manufacturing a conjugation product according to the preceding explanations. This method comprises the steps explained in the following. First, and antigen binding moiety is reacted with a compound that is able to covalently bound a cross-linker to the antigen binding moiety.

Afterwards, the antigen binding moiety comprising a cross-linker is reacted with a mucin binding moiety. As a result of this reaction step, the final conjugation product is formed, in which the antigen binding moiety and the mucin binding moiety are covalently linked to each other via the cross-linker.

In an embodiment, succinimidyl-4-(N-maleimidomethyl)cyclohexane-1-carboxylate (SMMC) is an appropriate compound that is able to covalently bound the cross-linker to the antigen binding moiety. The resulting cross-linker in the conjugation product can also be denoted as SMMC cross-linker.

The formed antigen binding moiety comprising a cross-linker can be purified prior to incubating it with the mucin binding moiety. Size exclusion chromatography (SEC) is a particularly appropriate method for such a purification step.

All embodiments of the conjugation product can be combined in any desired way and can be transferred either individually or in any arbitrary manner to the uses, to the medicament, and to the different methods. Likewise, all embodiments of the different uses can be combined in any desired way and can be transferred either individually or in any arbitrary manner to the conjugation product, to the respective other uses, to the medicament, and to the different methods. Likewise, all embodiments of the medicament can be combined in any desired way and can be transferred either individually or in any arbitrary manner to the conjugation product, to the different uses, and to the different methods. Furthermore, all embodiments of the different methods can be combined in any desired way and can be transferred either individually or in any arbitrary manner to the conjugation product, to the different uses, and to the respective other methods.

Further details of aspects of the present invention will be explained in the following making reference to exemplary embodiments and accompanying Figures. In the Figures:
- Figure 1: shows a high-resolution mass spectrum (HRMS) of imdevimab;
- Figure 2: shows a HRMS of a conjugation product of TFF3-p6 (SEQ ID NO. 67) and imdevimab;
- Figure 3: shows a HRMS of a conjugation product of TFF3 (SEQ ID NO. 65) and imdevimab;
- Figure 4: shows a HRMS of trastuzumab;
- Figure 5: shows a HRMS of a conjugation product of TFF3-p6 (SEQ ID NO. 67) and trastuzumab;
- Figure 6: shows a HRMS of a conjugation product of TFF3 (SEQ ID NO. 65) and imdevimab;
- Figure 7: shows the results of a first reducing SDS-PAGE for antibody conjugation analysis;
- Figure 8: shows the results of a second reducing SDS-PAGE for antibody conjugation analysis;
- Figure 9A: shows the results of a mass photometry (iSCAM) experiment conducted on imdevimab;
- Figure 9B: shows the results of an iSCAM experiment conducted on a conjugation product of TFF3-p6 (SEQ ID NO. 67) and imdevimab;
- Figure 10A: shows the results of an iSCAM experiment conducted on trastuzumab;
- Figure 10B: shows the results of an iSCAM experiment conducted on a conjugation product of TFF3-p6 (SEQ ID NO. 67) and trastuzumab;
- Figure 11: illustrates the SARS-CoV-2 (B.1 variant) plaque reduction in various samples 24 and 48 hours after treatment;
- Figure 12: illustrates the antiviral effect of TFF3-functionalized imdevimab having different functionalization degrees;
- Figure 13: illustrates the antiviral effect of a conjugation product of TFF3-p6 (SEQ ID NO. 67) and imdevimab; and
- Figure 14: illustrates the antiviral effect of conjugation products of TFF3-p6 (SEQ ID NO. 67) and imdevimab as well as of TFF3 (SEQ ID NO. 65) and imdevimab.

The Figures will be explained in the following in more detail in the context of exemplary embodiments.

### Cloning and production of TTF3-p6, and TFF3

TFF3(C57)-p6 and TFF3(C57) was recombinantly produced in *E.Coli* BL21-CodonPlus(DE3)-RIPL cells according to the protocol described below, which has been adapted from the protocol of Järvå *et al.,* 2020.

The gene fragment of human trefoil factor 3 (TFF3) with or without C-terminal p6 peptide, each comprising upstream periplasmatic secretion and affinity purification sequences (complete sequences: SEQ ID NO. 67 and SEQ ID NO. 65) was cloned via restriction cloning (Xbal and EcoRI) into the bacterial expression vector pET28b (called RD_GGS_TFF3(C57)p6 and RD_GG_TFF3(C57)). After heat shock transformation of the plasmid in *E.coli* BL21-CodonPlus(DE3)-RIPL cells, protein expression was conducted using TB AIM medium with selection antibiotics kanamycin and chloramphenicol. After 24 h at 250 rpm and ~25 °C, until a constant *E.coli* density (OD₆₀₀) was reached, cells were pelleted at 4,000 x g for 10 min. The pellet was washed twice with cold washing buffer (50 mM HEPES, 200 mM NaCl, 2,5 mM MgCl₂, 2,5 mM CaCl₂, pH 8.0, 4000 x g for 10 min), snap frozen in liquid nitrogen, and stored at -20 °C in aliquoted pellets each from 100 ml cell suspension until further utilization for lysis.

### Detergent-free cell lysis

The bacterial pellet was resuspended in 10 mL HisTag-binding buffer (300 mM NaCl, 50 mM TRIS, 40 mM imidazole, pH 7.5) containing additional 100 mM MgCl₂. The resuspension was treated with lysozyme (0.5 mg/mL end concentration) and DNase (0.05 mg/mL end concentration) and incubated for 30 minutes on ice. The cells were lysed by sonication (Bandelin Sonopuls HD 2020: 15 x 30 s, 90 %, 60 power) followed by centrifugation (12.000 G, 45 min, 4 °C). The supernatant was filtered (20 µM PES filter) and directly applied for FPLC purification.

### Affinity purification using FPLC

The obtained lysate was applied to a two-step purification protocol starting with a HisTag-purification. The sample was applied to an affinity column (HisTrap HP, 1 mL, Cytiva, Germany), washed with 10 column volumes of HisTag-binding buffer and eluted with 100 % of elution buffer (300 mM NaCl, 50 mM TRIS, 400 mM imidazole, pH 7.5). The eluted fraction was applied in a second purification step to a StrepTag column (Strep-Tactin XT 4Flow, 5 mL, IBA Lifesciences, Germany). After washing with 10 column volumes of binding buffer (100 mM TRIS, 150 mM NaCl, 1 mM EDTA, pH 8.0), the samples were eluted with 100 % of elution buffer (100 mM TRIS 150 mM NaCl, 1 mM EDTA, 50 mM Biotin, pH 8.0).

### Removal of affinity tags

The eluted fractions of the StrepTag purification were concentrated to yield a volume under 2 mL by ultracentrifugation (Amicon Ultra, 3 kDa CO, Merck, Germany). For cleavage of the purification tags, the sample was treated with TEV protease (one unit digests 15 µg overnight, ProTEV plus, Promega, Germany) and 20X ProTEV buffer (set 1X dilution, ProTEV plus, Promega, Germany). The reaction was incubated over night at 30 °C. To remove the cleaved tags and the HisTag-labelled protease, an additional FPLC HisTag-purification was performed. Imidazole was added to the sample to match the concentration of the binding buffer. The sample application was directly collected since the sample was tag-free and was found in the flow-through. The collected flow-through fractions were collected, concentrated and a buffer exchange to PBS pH 7.4 was accomplished by ultracentrifugation (Amicon Ultra, 3 kDa CO, Merck, Germany).

### Analytical quantification

For quantification of the sample, a spectrophotometer (NanoDrop 2000, Thermo Scientific, US) was utilized. The corresponding blank measurements for each sample were performed with the sample buffer. For protein quantification the measurements were performed at 280 nm. The purity was controlled by the OD 260/280 ratio indicating the DNA/protein ratio.

For mass spectrometric confirmation a UPLC-ESI-TOF-MS (Waters Synapt G2-S, Waters Zspray ESI Modul, US) equipped with a C4 column (Waters Acquity UPLC Protein BEH C4, US) was utilized. The injection volume was set to 7.5 µL with a flow rate of 0.2 mL/min. For chromatographic separation eluent A (95 %, water containing 0.1 % formic acid) and eluent B (5 %, acetonitrile containing 0.1 % formic acid) were initially hold for 2 minutes, followed by increase/decrease to 5 % of eluent A and 95 % of eluent B in 23 minutes.

### Production and characterization of TFF3-coniuaated antibodies

### Production of Imdevimab-TFF3p6 conjugate

imdevimab was provided as a PBS solution (1.04 mg/ml, 7.14 µM). TFF3p6 was used directly from a PBS solution (28.5 µM).
1. Initially, 90 µl of imdevimab (0.64 nmol) were incubated with succinimidyl-4-(N-maleimidomethyl)cyclohexane-1-carboxylate crosslinker (SMCC, 25 eq, 16 nmol), previously dissolved in the minimal possible volume of DMSO. The pH was adjusted to 7.2 and the reaction mixture was kept at room temperature, with constant stirring, for 30 min.
2. Subsequently, the excess of non-reacted SMCC was removed using a 0.5 ml Zeba Spin desalting column.
3. Next, 275 µl of TFF3p6 (12.5 eq, 7.8 nmol) were added to the mixture, the pH was adjusted to 7.2, and incubation followed at room temperature for additional 30 min. This incubation resulted in a conjugation of the cysteine at position 57 (not counting any N-terminal amino acids of the sortase acceptor site/solubility tag) to amines of lysins on imdevimab using the SMCC linker.
4. Immediate purification followed by adjusting the volume of the reaction to 400 µl, centrifuged at 16000 rcf for 5 min, filtered, and injected into a Bio-rad NGC Quest Plus System for Size Exclusion Chromatography purification, using a Superose^{®} 6 Increase 10/300 GL column. Fractions with protein and free of unreacted TFF3p6 and SMCC, were collected, and the volume was decrease to 0.125 ml using a Satorious Vivaspin^{®} 500 Centrifugal Concentrator.
5. The final concentration of the sample was determined using a NanoDrop One/Onec UV-VIS Spectrophotometer, yielding 1.46 µM. Subsequent analysis followed by SDS-page HRMS.

High resolution ESI-MS spectra (HRMS) were recorded on a Waters H-class instrument equipped with a quaternary solvent manager, a Waters sample manager-FTN, a Waters PDA detector and a Waters column manager with an Acquity UPLC protein BEH C18 column (1.7 µm, 2.1 mm x 50 mm). Samples were eluted with a flow rate of 0.3 mL/min. The following gradient was used: A: 0.01 % FA in H2O; B: 0.01 % FA in MeCN. 5 % B: 0-1 min; 5 to 95 % B: 1-7min; 95 % B: 7 to 8.5 min. Mass analysis was conducted with a Waters XEVO G2-XS QTof analyzer.

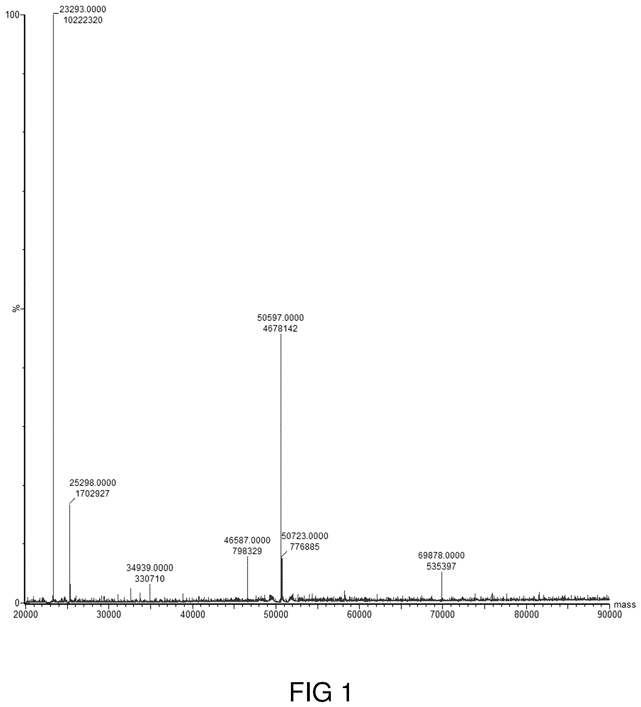

Figure 1 shows a high-resolution mass spectrum of non-conjugated imdevimab that was recorded as described in the preceding paragraph. The observed signals can be assigned as follows (light chain - LC; heavy chain - HC): LC, 23293 Da; HC/2, 25298 Da; 2LC, 46587 Da; HC, 50597 Da

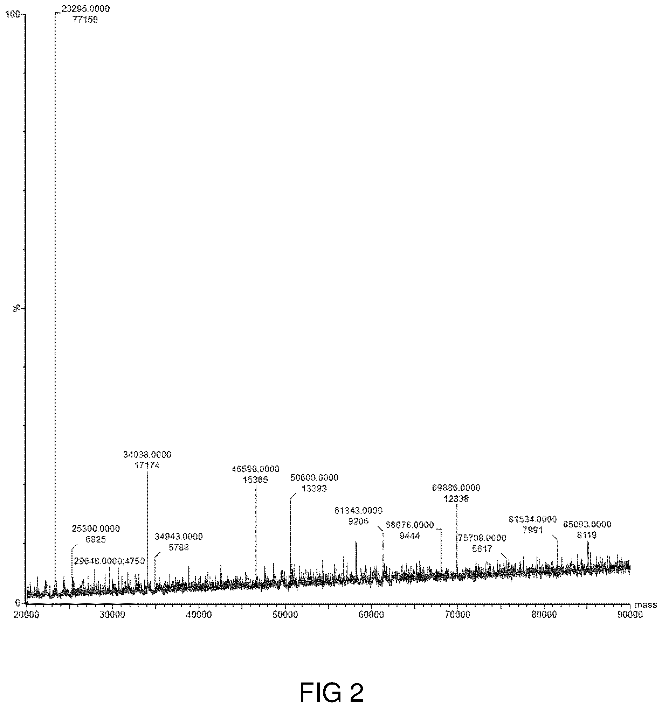

Figure 2 shows a high-resolution mass spectrum of imdevimab conjugated with TFF3-p6. The observed signals can be assigned as follows: LC, 23295 Da; LC + TFF3p6, calculated: 34040 Da, experimental: 34038 Da; HC, 50600 Da; HC + TFF3p6, calculated: 61344 Da, experimental: 61343 Da

### Production of Imdevimab-TFF3 conjugate

imdevimab, was provided as a PBS solution (1.04 mg/ml, 7.14 µM). TFF3 was used directly from a PBS solution (37.6 µM).

Steps 1, 2, 4, and 5 of the production of the lmdevimab-TFF3p6 conjugate described before were also done here. In step 3, 202 µl of TFF3 (12.5 eq, 7.6 nmol) were added to the mixture, the pH was adjusted to 7.2, and incubation followed at room temperature for additional 30 min. In step 5, a final concentration of 2.36 µM was determined.

HRMS were recorded as described before for the production of the lmdevimab-TFF3p6 conjugate.

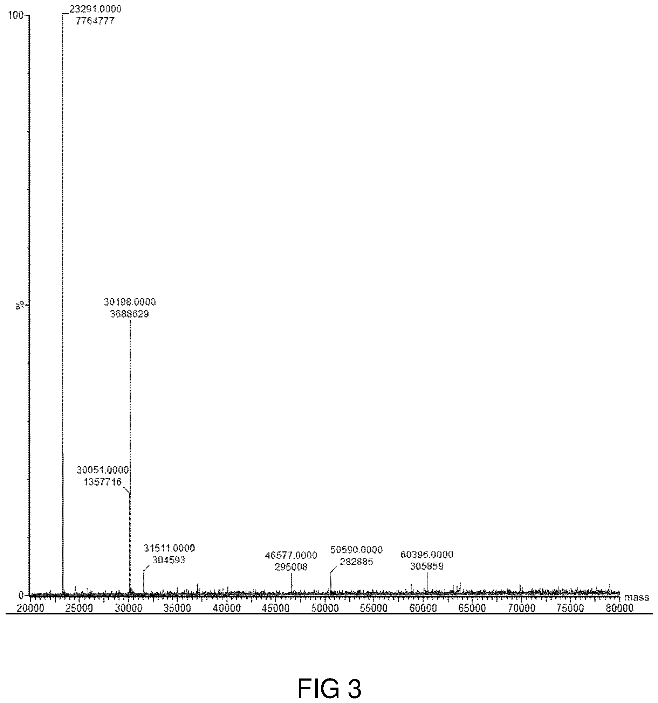

Figure 3 shows a high-resolution mass spectrum of imdevimab conjugated with TFF3. The observed signals can be assigned as follows: LC, 23291 Da; LC+TFF3, calculated: 30198 Da, experimental 30198 Da; 2LC, 46577 Da; HC, 50590 Da; 2[LC+TFF3], 60396 Da)

### Production of Trastuzumab-TFF3p6 conjugate

Trastuzumab was provided as a PBS solution (1.0 mg/ml, 6.87 µM). TFF3p6 was used directly from a PBS solution (28.5 µM).
1. Initially, 90 µl of Trastuzumab (0.61 nmol) were incubated with succinimidyl-4-(N-maleimidomethyl)cyclohexane-1-carboxylate crosslinker (SMCC, 25 eq, 15.4 nmol), previously dissolved in the minimal possible volume of DMSO. The pH was adjusted to 7.2 and the reaction mixture was kept at room temperature, with constant stirring, for 30 min.
2. Subsequently, the excess of non-reacted SMCC was removed using a 0.5 ml Zeba Spin desalting column.
3. Next, 267 µl of TFF3p6 (12.5 eq, 7.6 nmol) were added to the mixture, the pH was adjusted to 7.2, and incubation followed at room temperature for additional 30 min. This incubation resulted in a conjugation of the cysteine at position 57 (not counting any N-terminal amino acids of the sortase acceptor site/solubility tag) to amines of lysins on trastuzumab using the SMCC linker.
4. Immediate purification followed by adjusting the volume of the reaction to 400 µl, centrifuged at 16000 rcf for 5 min, filtered, and injected into a Bio-rad NGC Quest Plus System for Size Exclusion Chromatography purification, using a Superose^{®} 6 Increase 10/300 GL column. Fractions with protein and free of unreacted TFF3p6 and SMCC, were collected, and the volume was decrease to 0.125 ml using a Satorious Vivaspin^{®} 500 Centrifugal Concentrator.
5. The final concentration of the sample was determined using a NanoDrop One/Onec UV-VIS Spectrophotometer, yielding 2.4 µM. Subsequent analysis followed by SDS-page HRMS.

HRMS were recorded as described before for the production of the lmdevimab-TFF3p6 conjugate.

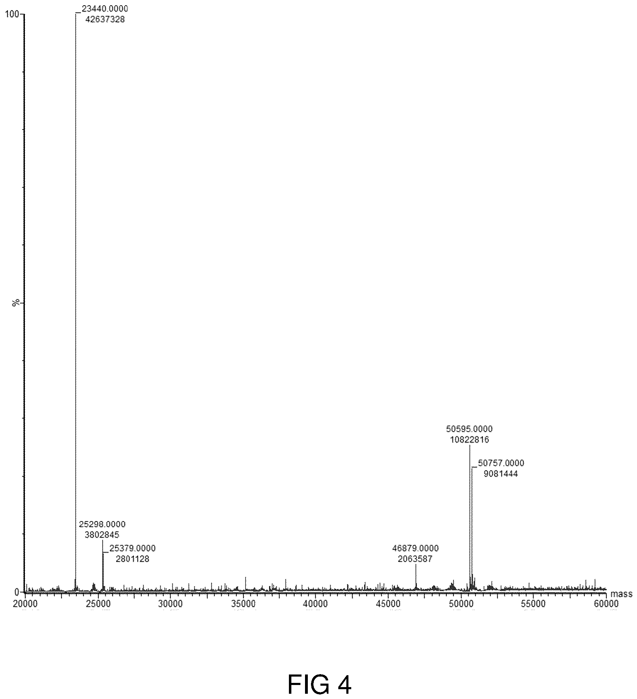

Figure 4 shows a high-resolution mass spectrum of non-conjugated trastuzumab that was recorded as described above. The observed signals can be assigned as follows: LC, 23440 Da; HC/2, 25298 Da, 25379 Da; 2LC, 46879 Da; HC, 50595 Da, 50757 Da

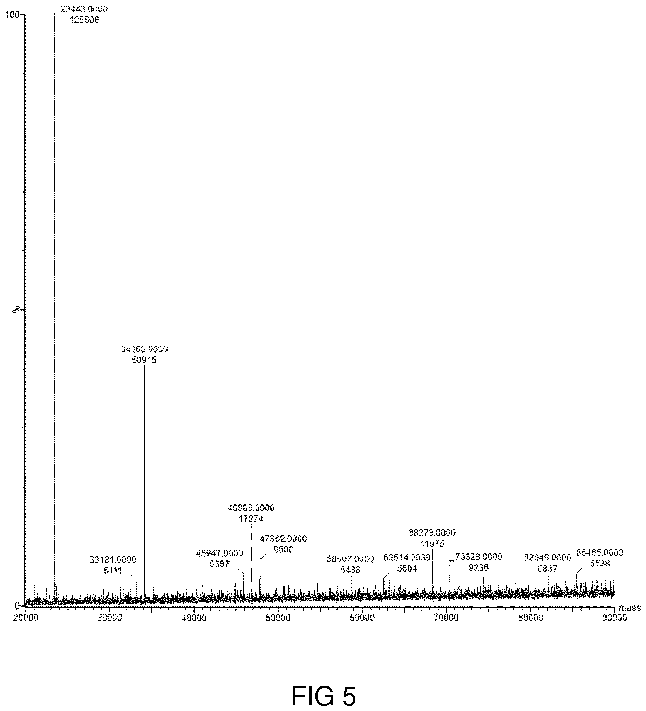

Figure 5 shows a high-resolution mass spectrum of trastuzumab conjugated with TFF3-p6. The observed signals can be assigned as follows: LC, 23443 Da; LC + TFF3p6, calculated: 34187, experimental: 34186

### Production of Trastuzumab-TFF3 conjugate

Trastuzumab was provided as a PBS solution (1.0 mg/ml, 6.87 µM). TFF3 was used directly from a PBS solution (37.6 µM).

Steps 1, 2, 4, and 5 of the production of the Trastuzumab-TFF3p6 conjugate described before were also done here. In step 3, 202 µl of TFF3 (12.5 eq, 7.6 nmol) were added to the mixture, the pH was adjusted to 7.2, and incubation followed at room temperature for additional 30 min. In step 5, a final concentration of 3.05 µM was determined.

HRMS were recorded as described before for the production of the lmdevimab-TFF3p6 conjugate.

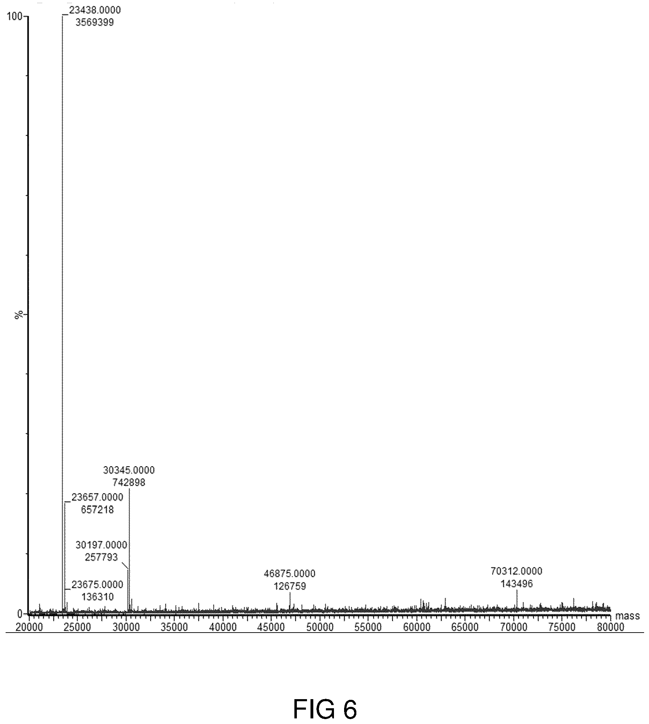

Figure 6 shows a high-resolution mass spectrum of trastuzumab conjugated with TFF3-p6. The observed signals can be assigned as follows: LC, 23438 Da; LC+TFF3, calculated, 30345 Da, experimental, 30345 Da; 2LC, 46875 Da; 3LC 70312 Da

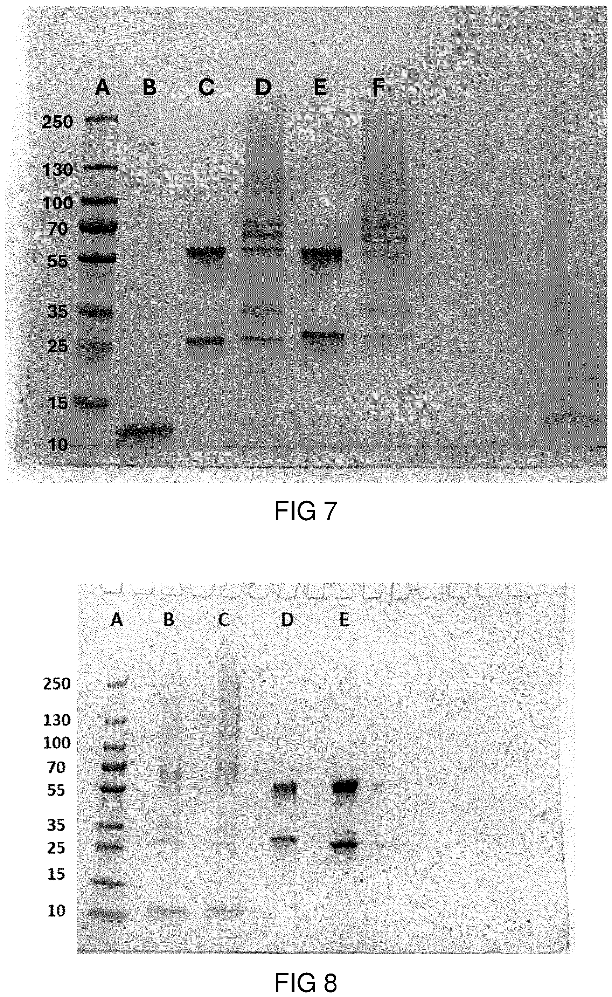

Figure 7 shows the results of a first reducing SDS-PAGE for antibody conjugation analysis. Lane A: Ladder; lane B: TFF3p6 (10.5 kDa); lane C: Trastuzumab (LC, 23.4 kDa; HC, 50.5 kDa); lane D: Trastuzumab-TFF3p6 (LC, 23.4 kDa; LC + TFF3p6, 34.2 kDa; LC + 2TFF3p6, 44.9 kDa), (HC, 50.6 kDa; HC + TFF3p6, 61.3 kDa; HC + 2TFF3p6, 72.0 kDa); lane E: imdevimab (LC, 23.3 kDa; HC, 50.6 kDa); lane F: Imdevimab-TFF3p6 (LC, 23.3 kDa; LC + TFF3p6, 34.0 kDa; LC + 2TFF3p6, 44.7 kDa), (HC, 50.5 kDa; HC + TFF3p6, 61.3 kDa; HC + 2TFF3p6, 72.0 kDa). The success of conjugating TFF3-p6 with trastuzumab or imdevimab, respectively, can clearly be seen from this SDS-PAGE.

Figure 8 shows the results of a second reducing SDS-PAGE for antibody conjugation analysis. Lane A: Ladder; lane B: lmdevimab-TFF3 (LC, 23291 Da; LC+TFF3, 30198 Da; HC, 50590 Da; HC+TFF3, 57497 Da); lane C: Trastuzumab-TFF3 (LC, 23438 Da; LC+TFF3, 30345 Da; HC, 50500 Da; HC+TFF3, 57407 Da); lane D: imdevimab (LC, 23.3 kDa; HC, 50.6 kDa); lane E: Trastuzumab (LC, 23.4 kDa; HC, 50.5 kDa). The success of conjugating TFF3 with trastuzumab or imdevimab, respectively, can clearly be seen from this SDS-PAGE.

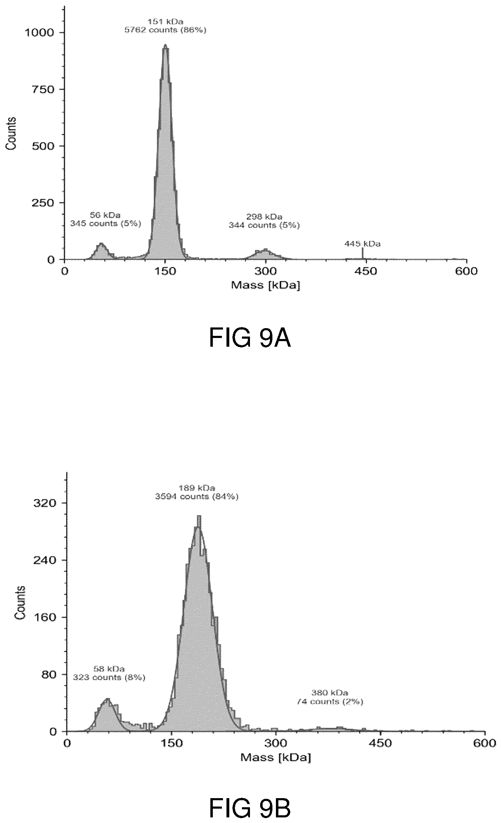

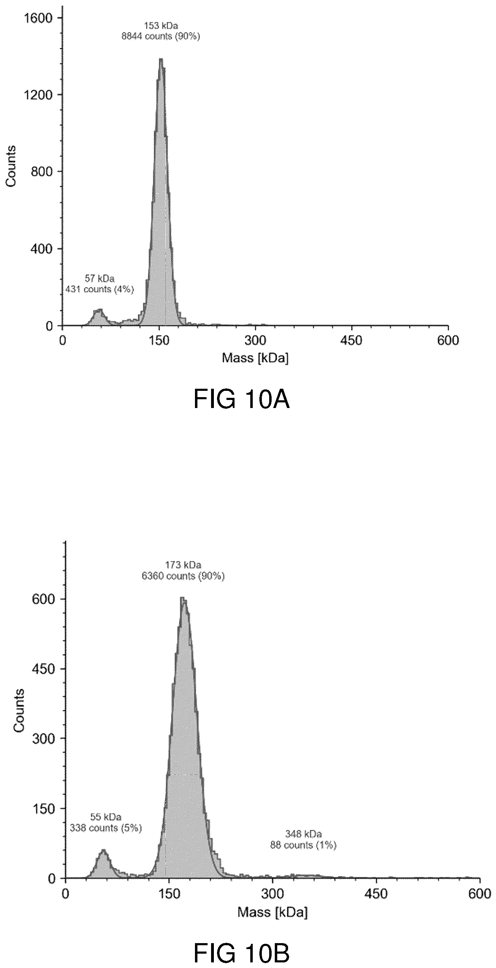

Figures 9A, 9B, 10A, and 10B show the results of a mass shift analysis upon conjugation of imdevimab or trastuzumab to TFF3-p6 using mass photometry (iSCAM). It can well be seen that the molecular mass of imdevimab (Figure 9A) is shifted towards higher values upon conjugation with TFF3-p6 (Figure 9B). Likewise, it can well be seen that the molecular mass of trastuzumab (Figure 10A) is shifted towards higher values upon conjugation with TFF3-p6 (Figure 10B). The mean amount of conjugated TFF3-p6 to the antibodies was calculated from literature values for the molecular weight of the antibodies and a mass spectrometric analysis of TFF3-p6. The results are summarized in the following Table 6.

**Table 6: Results of mass shift analysis.**

| **Compound** | **Expected molecular weight / kDa** | **Molecular weight determined by iSCAM / kDa** | **Approximate amount of conjugated TFF3-p6 molecules per antibody determined from mass shift** |
|---|---|---|---|
| TFF3-p6 (with SMCC cross-linker) | - | 10.8 | - |
| Imdevimab | 144 | 151 | - |
| Imdevimab-(TFF3-p6)ₙ | - | 189 | 3.5 |
| Trastuzumab | 148 | 153 | - |
| Trastuzumab-(TFF3-p6)ₙ | - | 173 | 1.8 |

### In vitro infection inhibition studies

### Working virus preparation

A SARS-CoV-2 virus stock was thawed and diluted to a final concentration of 2500 pfu/ml using DMEM: F12 1:1 + GlutaMax (Gibco, Ref: 31331-028) containing 10 % fetal bovine serum, 100 IU penicillin G and 100 µg streptomycin, as well as 1 % non-essential amino acids.

### Compound preparation

The monoclonal antibody (imdevimab) was diluted in PBS to a final concentration of 2.5 µg/ml (=0.25 µg/100 µl/well). Total amounts of the other compounds were calculated according to their molar mass such a way that equimolar amounts of all compounds were used.

### Monolayer infection

### Cells

CaLu-3 cells were seeded in 6-well plates and grown to a confluency of 90-100 %.

### Protocol

- Compounds were plated in triplicates on a 48-well plate (100 µl/well).
- 400 µl (= 1000 pfu/well) of working virus were added to each well and incubated for 45 minutes at room temperature.
- Medium was removed from 6-well plates containing confluent CaLu-3 cells.
- 500 µl of working virus/compound mix were transferred to each well and incubated for 1 hour at 37 °C and 5 % CO2.
- Cells were washed with PBS; 2 ml of CaLu-3 medium were added per well.
- 24 and 48 hours after infection, 1mL supernatant was collected from each well and frozen at -80 °C until titration.
- A cell and a virus control were included in every assay.

### Monolayer infection

### Air liquid interface (ALI) cultures

Calu-3 cells were seeded on the apical side of a 6-well plate with 1mL culture medium; 1.6mL culture medium was added into the basolateral side. After culture for 7 days, medium was removed from the apical side to obtain the ALI conditions. The basolateral medium was refreshed every 2-3 days and the infection was performed at the 7th day of ALI culture.

### Protocol

- Compounds were plated in triplicates on a 48-well plate (100 µl/well).
- 400 µl (= 1000 pfu/well) of working virus were added to each well and incubated for 45 minutes at room temperature.
- Medium was removed from the basolateral side of 6-well plates containing confluent CaLu-3 cells.
- 500 µl of working virus/compound mix were transferred to the apical side of each well and incubated for 1 hour at 37 °C and 5 % CO2.
- Cells were washed with PBS; 1.6 ml of CaLu-3 medium were added to the basolateral side per well.
- 24 after infection 500 µL supernatant was collected from each well, and 500 mL fresh medium were added into the basolateral side. 48 h after infection all supernatant was collected from each well and frozen at -80 °C until titration
- A cell and a virus control were included in every assay.

### Plague assay

### Cells

Vero E6 cells were seeded in 12- well plates and grown to a confluency of 80 %.

### Protocol

- Cell culture supernatant stocks from 24- and 48-hour time points were thawed.
- 10-fold dilutions (-1 to -6) in MEM (minimal essential medium) containing 1 % fetal bovine serum, 100 IU penicillin G and 100 µg streptomycin were prepared and plated on Vero E6 cells.
- After 1 hour and 15 min of incubation at 37 °C and 5 % CO2, supernatant was removed and cells were overlaid with 2X Eagle's Minimum Essential Medium (EMEM; Lonza^{™} BioWhittaker^{™}) medium containing 1.5 % microcrystalline cellulose and carboxymethyl cellulose sodium (Vivapur 611p; JRS Pharma).
- Cells were incubated for 48 hours at 37 °C and 5 % CO2 and fixed with 4 % PBS-buffered formaldehyde.
- To visualize the plaques, plates were stained with 0.75 % methylene blue.

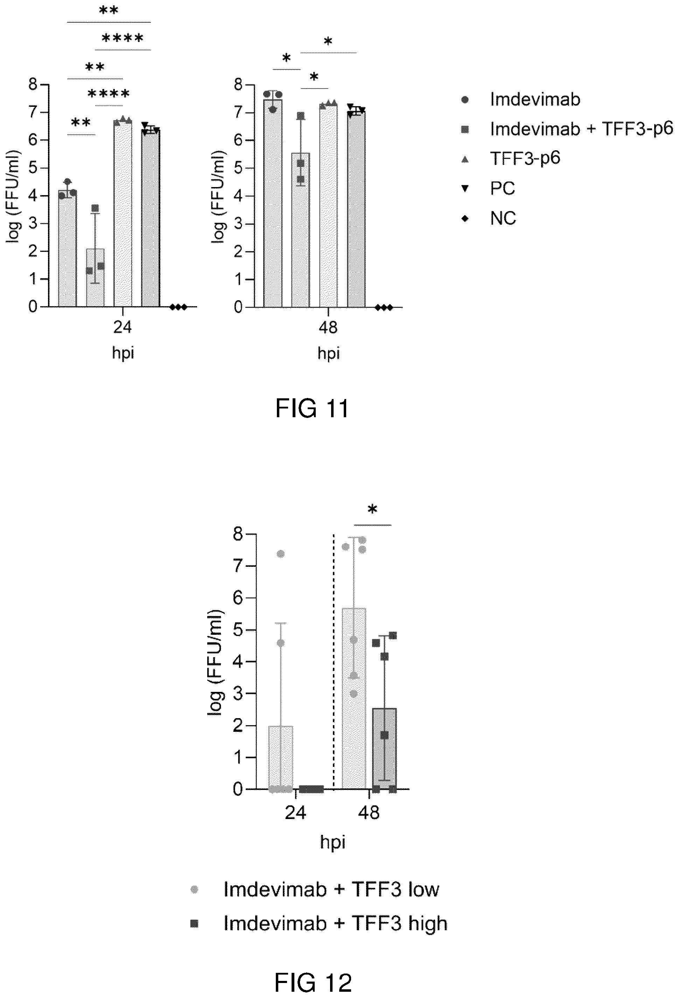

Figure 11 illustrates the SARS-CoV-2 (B.1 variant) plaque reduction in various samples 24 and 48 hours after treatment. The data was generated using highly confluent, mucus covered, submerged Calu-3 cell cultures infected with 1000 pfu SARS-CoV-2 (B.1 variant) previously incubated for 45 minutes with either 1.7 nM imdevimab, 1.7 nM Imdevimab-TFF3-p6 or 1.7 nM TFF3-p6 in PBS. 1000 pfu of SARS-CoV-2 incubated for 45 minutes with an equal volume of PBS served as positive control (PC). Non-infected, non-treated Calu-3 cells served as negative control (NC). The experiment was replicated three times independently, data points indicate the mean value of duplicate titrations on Vero E6 cells. Parametric statistics on log transformed data, ordinary one-way ANOVA with Tukey`s multiple comparisons test. *p < 0.05, **p < 0.01, and ****p < 0.0001. It can clearly be seen that the plaque reduction of the conjugation product of imdevimab and TFF3-p6 is significantly better than the plaque reduction of the pure antibody imdevimab. In addition, the observed effect lasts longer. While the plaque reduction of pure imdevimab can only be seen at 24 hours after infection, the conjugation product of imdevimab and TFF3-p6 efficiently reduces plaques also 48 hours after infection.

Figure 12 illustrates the antiviral effect of TFF3-functionalized imdevimab having different functionalization degrees. In this comparison of imdevimab-TFF3 batches with different degrees of functionalization, the same procedures were applied as described in Figure 11. "TFF3 low" denotes one molecule of imdevimab coupled to ~2.7 molecules of TFF3. "TFF3 high" denotes one molecule of imdevimab coupled to ~3.5 molecules of TFF3. Parametric statistics was done on log transformed data; an unpaired t test with Welch correction was performed. *p < 0.05. a higher amount of TFF 3 molecules per antibody results in a higher virus binding efficacy and thus a more prominent antiviral effect. Thus, it is possible to tune the antiviral efficacy of imdevimab and thus the anti-pathogenic efficacy of other antibodies by increasing the number of mucin binding moieties in the produced conjugation product, i.e., by increasing the ratio between mucin binding moieties and antigen binding moieties in the conjugation product.

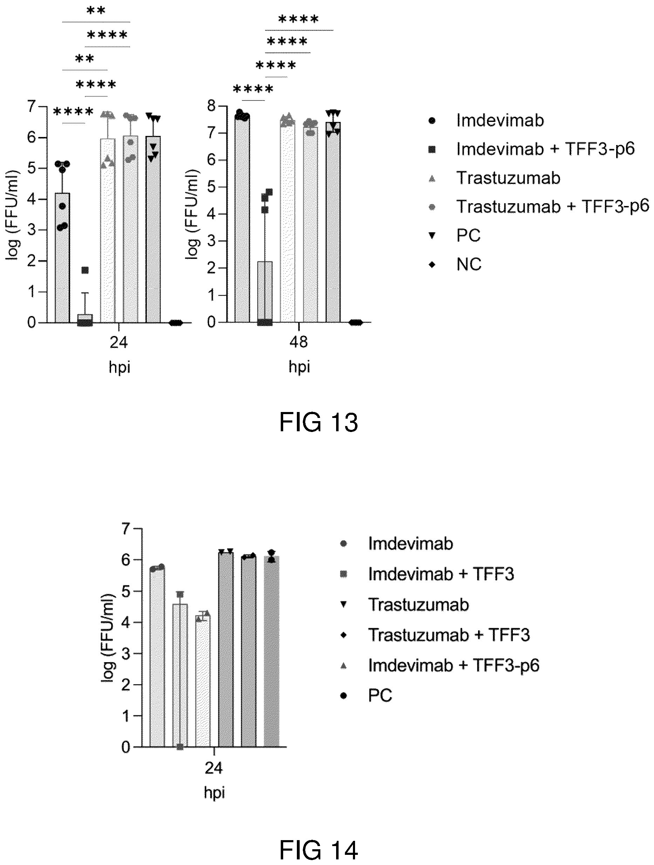

Figure 13 illustrates the antiviral effect of a conjugation product of TFF3-p6 (SEQ ID NO. 67) and imdevimab and compares it with the antiviral effect of non-conjugated imdevimab as well as of trastuzumab. Trastuzumab is an anti-breast cancer antibody that does not bind SARS-CoV-2. It serves here as isotype control. The data was generated by titration of a Calu-3 cell culture supernatant on Vero E6 cells. The positive control (PC) is virus infection in absence of any inhibitor, the negative control (NC) are cells in absence of virus infection. Parametric statistics was done on log transformed data; an ordinary one-way ANOVA with Tukey`s multiple comparisons test was performed. *p < 0.05, **p < 0.01, and ****p < 0.0001. It can well be seen that the antiviral efficacy of imdevimab is significantly increased by conjugating it to TFF3-p6 (both at 24 hours after infection and at 48 hours after infection). In addition, the conjugation of TFF3-p6 does not have any relevant effect on the antiviral efficacy of trastuzumab. Neither in it pure form nor after conjugation with TFF3-p6, trastuzumab is able to bind any SARS-CoV-2. Thus, the observed efficacy enhancement of imdevimab is not due to an inherent antiviral property of TFF3-p6, but is rather due to the conjugation of imdevimab to TFF3-p6.

Figure 14 illustrates the antiviral effect of conjugation products of TFF3-p6 (SEQ ID NO. 67) and imdevimab as well as of TFF3 (SEQ ID NO. 65) and imdevimab, wherein unconjugated imdevimab serves as control and trastuzumab serves as isotype control. The data was generated by titration of Calu-3 cell culture supernatant on Vero E6 cells. The positive control (PC) was virus infection in the absence of inhibitor. The antiviral effect of imdevimab-TFF3 does not significantly deviate from the antiviral effect of imdevimab-TFF3-p6. however, both conjugation products shows significantly better antiviral efficacy than pure (non-conjugated) imdevimab, which still has a better antiviral effect than the positive control or trastuzumab or the conjugation product trastuzumab-TFF3. Thus, it does not play a relevant role whether the mucin binding moiety has itself antiviral properties (as in case of TFF3-p6) or not (as in case of TFF3). Rather, the presence of any mucin binding moiety is relevant for enhancing the antiviral effect of imdevimab or, consequently, the clearing effect against antigens of the antigen binding moiety, in particular the anti-pathogenic effect of any antibody used as antigen binding moiety.

### List of references cited in the preceding sections or otherwise considered relevant

1. Bhatia, Sumati, et al. "Linear polysialoside outperforms dendritic analogs for inhibition of influenza virus infection in vitro and in vivo." Biomaterials 138 (2017): 22-34.
2. Braga Emidio, Nayara, et al. "Chemical Synthesis of TFF3 Reveals Novel Mechanistic Insights and a Gut-Stable Metabolite." Journal of Medicinal Chemistry 64.13 (2021): 9484-9495.
3. Bui, Vuong N., et al. "Pathogenicity of an H5N1 avian influenza virus isolated in Vietnam in 2012 and reliability of conjunctival samples for diagnosis of infection." Virus Research 179 (2014): 125-132.
4. Cabán, Madelyn, et al. "Cross-protective antibodies against common endemic respiratory viruses." Nature Communications 14.1 (2023): 798.
5. Han, Dong P., Adam Penn-Nicholson, and Michael W. Cho. "Identification of critical determinants on ACE2 for SARS-CoV entry and development of a potent entry inhibitor." Virology 350.1 (2006): 15-25.
6. Hoffmann, Markus, et al. "SARS-CoV-2 cell entry depends on ACE2 and TMPRSS2 and is blocked by a clinically proven protease inhibitor." Cell 181.2 (2020): 271-280.
7. Hoffmann, Miriam, Nicole L. Snyder, and Laura Hartmann. "Polymers Inspired by Heparin and Heparan Sulfate for Viral Targeting." Macromolecules 55.18 (2022): 7957-7973.
8. Hoffmann, Werner. "Trefoil factor family (TFF) peptides and their different roles in the mucosal innate immune defense and more: An update." Current Medicinal Chemistry 28.36 (2021): 7387-7399.
9. Hrebik, Dominik, et al. "ICAM-1 induced rearrangements of capsid and genome prime rhinovirus 14 for activation and uncoating." Proceedings of the National Academy of Sciences 118.19 (2021): e2024251118.
10. Issmail, Leila, et al. "Prefusion-specific antibody-derived peptides trivalently presented on DNA-nanoscaffolds as an innovative strategy against RSV entry." Frontiers in Virology 2 (2022).
11. Järvå, Michael A., et al. "Trefoil factors share a lectin activity that defines their role in mucus." Nature Communications 11.1 (2020): 1-9.
12. Jerabek-Willemsen M., et al. "MicroScale Thermophoresis: Interaction analysis and beyond." Journal of Molecular Structure 1077 (2014): 101-113.
13. Khan, Abdul Ghafoor, et al. "Human rhinovirus type 54 infection via heparan sulfate is less efficient and strictly dependent on low endosomal pH." Journal of Virology 81.9 (2007): 4625-4632.
14. Madsen, Anders, et al. "Human antibodies targeting influenza B virus neuraminidase active site are broadly protective." Immunity 53.4 (2020): 852-863.
15. McLellan, Jason S., et al. "Structure-based design of a fusion glycoprotein vaccine for respiratory syncytial virus." science 342.6158 (2013): 592-598.
16. Memczak, Henry, et al. "Anti-hemagglutinin antibody derived lead peptides for inhibitors of influenza virus binding." PLoS One 11.7 (2016): e0159074.
17. Sarto, Carolina, et al. "Atomistic insight into the essential binding event of ACE2-derived peptides to the SARS-CoV-2 spike protein." Biological Chemistry 403.5-6 (2022): 615-624.
18. Smith, Thomas J., et al. "Neutralizing antibody to human rhinovirus 14 penetrates the receptor-binding canyon." Nature 383.6598 (1996): 350-354.
19. Thim, L., F. Madsen, and S. S. Poulsen. "Effect of trefoil factors on the viscoelastic properties of mucus gels." European Journal of Clinical Investigation 32.7 (2002): 519-527.
20. Zhu, Oing, et al. "A highly potent extended half-life antibody as a potential RSV vaccine surrogate for all infants." Science Translational Medicine 9.388 (2017): eaaj1928.
21. Zuo, Teng, et al. "Comprehensive analysis of antibody recognition in convalescent humans from highly pathogenic avian influenza H5N1 infection." Nature Communications 6.1 (2015): 8855.
22. Swanson, Michael D., et al. "Engineering a therapeutic lectin by uncoupling mitogenicity from antiviral activity." Cell 163.3 (2015): 746-758.
23. Mazalovska, Milena, and J. Calvin Kouokam. "Lectins as promising therapeutics for the prevention and treatment of HIV and other potential coinfections." BioMed Research International 2018.1 (2018): 3750646.
24. Schnider, Boris, et al. "HumanLectome, an update of UniLectin for the annotation and prediction of human lectins." Nucleic Acids Research 52.D1 (2024): D1683-D1693.

## Claims

1. Conjugation product comprising an antigen binding moiety and a mucin binding moiety covalently bound to the antigen binding moiety, with the proviso that the antigen binding moiety is neither an IgG-Fc-binding protein (FCGB) nor, if the antigen binding moiety is a peptide having less than 50 amino acids, a virus binding moiety that comprises or is a peptide being at least 95 % identical to SEQ ID NO. 2, SEQ ID NO. 3, SEQ ID NO. 4, SEQ ID NO. 5, SEQ ID NO. 6, SEQ ID NO. 7, SEQ ID NO. 8, SEQ ID NO. 9, SEQ ID NO. 10, SEQ ID NO. 11, SEQ ID NO. 12, SEQ ID NO. 13, SEQ ID NO. 23, SEQ ID NO. 24, SEQ ID NO. 25, SEQ ID NO. 26, SEQ ID NO. 27, SEQ ID NO. 28, SEQ ID NO. 29, SEQ ID NO. 30, SEQ ID NO. 31, SEQ ID NO. 32, SEQ ID NO. 33, SEQ ID NO. 34, SEQ ID NO. 35, SEQ ID NO. 36, SEQ ID NO. 37, SEQ ID NO. 38, SEQ ID NO. 39, SEQ ID NO. 40, SEQ ID NO. 41, SEQ ID NO. 42, SEQ ID NO. 43, SEQ ID NO. 48, SEQ ID NO. 49, SEQ ID NO. 50, SEQ ID NO. 51, SEQ ID NO. 52, SEQ ID NO. 53, SEQ ID NO. 54, SEQ ID NO. 55, SEQ ID NO. 56, SEQ ID NO. 57, SEQ ID NO. 58, SEQ ID NO. 59, SEQ ID NO. 60, SEQ ID NO. 61, SEQ ID NO. 62, or SEQ ID NO. 63.

2. Conjugation product according to claim 1, **characterized in that** the mucin binding moiety is chosen from the group consisting of polycarbophil, gelatin, hydroxypropyl methylcellulose, mucoadhesive cellulose based materials, cationic cellulose derivatives, chitosan, thiolated derivatives of polymers, polysulfates, polyacrylates, poloxamers, polysaccharides, sucralfate, maleimide-functionalized polymers for thiol-binding, guar gum, porcine gastric mucins, polyvinyl alcohol, and moieties comprising or being a peptide or a protein being at least 95 % identical to SEQ ID NO. 1, SEQ ID NO. 14, SEQ ID NO. 18, SEQ ID NO. 19, SEQ ID NO. 20, SEQ ID NO. 21, SEQ ID NO. 22, SEQ ID NO. 64, SEQ ID NO. 65, SEQ ID NO. 66, SEQ ID NO. 67, SEQ ID NO. 68, SEQ ID NO. 69, SEQ ID NO. 70, SEQ ID NO. 71, SEQ ID NO. 72, SEQ ID NO. 73, SEQ ID NO. 74, SEQ ID NO. 75, SEQ ID NO. 76, SEQ ID NO. 77, SEQ ID NO. 78, SEQ ID NO. 79, SEQ ID NO. 80, SEQ ID NO. 81, SEQ ID NO. 82, SEQ ID NO. 83, SEQ ID NO. 84, SEQ ID NO. 85, SEQ ID NO. 86, SEQ ID NO. 87, SEQ ID NO. 88, SEQ ID NO. 89, SEQ ID NO. 90, SEQ ID NO. 91, SEQ ID NO. 92, SEQ ID NO. 93, SEQ ID NO. 94, SEQ ID NO. 95, SEQ ID NO. 96, SEQ ID NO. 97, SEQ ID NO. 98, SEQ ID NO. 99, SEQ ID NO. 100, SEQ ID NO. 101, SEQ ID NO. 102, SEQ ID NO. 103, SEQ ID NO. 104, SEQ ID NO. 105, SEQ ID NO. 106, SEQ ID NO. 107, SEQ ID NO. 108, SEQ ID NO. 109, SEQ ID NO. 110, SEQ ID NO. 111, SEQ ID NO. 112, SEQ ID NO. 113, SEQ ID NO. 114, SEQ ID NO. 115, SEQ ID NO. 116, SEQ ID NO. 117, SEQ ID NO. 118, SEQ ID NO. 119, SEQ ID NO. 120, SEQ ID NO. 121, SEQ ID NO. 122, SEQ ID NO. 123, SEQ ID NO. 124, SEQ ID NO. 125, SEQ ID NO. 126, SEQ ID NO. 127, SEQ ID NO. 128, SEQ ID NO. 129, SEQ ID NO. 130, SEQ ID NO. 131, SEQ ID NO. 132, SEQ ID NO. 133, SEQ ID NO. 134, SEQ ID NO. 135, SEQ ID NO. 136, or SEQ ID NO. 137.

3. Conjugation product according to claim 1 or 2, **characterized in that** mucin binding moiety comprises or is a peptide being at least 95 % identical to SEQ ID NO. 1, SEQ ID NO. 14, SEQ ID NO. 20, SEQ ID NO. 21, SEQ ID NO. 22, SEQ ID NO. 64, SEQ ID NO. 65, SEQ ID NO. 66, SEQ ID NO. 67, SEQ ID NO. 68, SEQ ID NO. 69, or SEQ ID NO. 70.

4. Conjugation product according to any of the preceding claims, **characterized in that** the mucin binding moiety comprises or is a trefoil factor 3 peptide being at least 95 % identical to SEQ ID NO. 14.

5. Conjugation product according to any of the preceding claims, **characterized in that** the antigen binding moiety is capable of binding a virus, a bacterium, an allergen, or a sperm cell.

6. Conjugation product according to any of the preceding claims, **characterized in that** the antigen binding moiety comprises or is a paratope, a foldamer, or a polysulfate.

7. Conjugation product according to claim 6, **characterized in that** the antigen binding moiety comprises a paratope covalently bound to a scaffold protein or a scaffold polymer.

8. Conjugation product according to claim 6 or 7, **characterized in that** the antigen binding moiety comprises or is a paratope of an antiviral antibody, of an antibacterial antibody, of an anti-allergen antibody, or of an anti-sperm cell antibody.

9. Conjugation product according to claim 8, **characterized in that** the antigen binding moiety is an antiviral antibody, an antibacterial antibody, an anti-allergen antibody, or an anti-sperm cell antibody.

10. Conjugation product according to claim 8 or 9, **characterized in that** the antiviral antibody is an antibody targeting at least one of SARS-CoV-1, SARS-CoV-2, MERS-CoV, influenza A virus, influenza A/B virus, respiratory syncytial virus, human parainfluenza virus, rhinovirus, orthopoxvirus, herpes simplex virus, hepatitis B virus, hepatitis C virus, varicella zoster virus, canine parvovirus, cytomegalovirus, and human immunodeficiency virus.

11. Conjugation product according to any of claims 8 to 10, **characterized in that** the antiviral antibody is chosen from the group consisting of REGN10989, REGN10986, REGN10934, REGN10964, REGN10954, REGN10984, adintrevimab, amubarvimab, bamlanivimab, bebtelovimab, beludavimab, canrivitug, casirivimab, cilgavimab, crexavibart, ensovibep, enuzovimab, etesevimab, gorivitug, imdevimab, lomtegovimab, masavibart, nepuvibart, nisfevitug, ogalvibart, pemivibart, plutavimab, regdanvimab, rimteravimab, romlusevimab, simaravibart, sipavibart, sotrovimab, timcevibart, tixagevimab, upanovimab, CR3014, m396, REGN3051, REGN3048, m336, LCA60, 4C2, 2E6, 3B11-N, MERS-4, MERS-27, firivumab, gedivumab, navivumab, CR9114, VIS410, FI6, MEDI8852, VIR-2482, MHAA4549A, 2G02, FNI9, FI6v3, clesrovimab, felvizumab, gontivimab, motavizumab, nirsevimab, palivizumab, suptavumab, HNK20, RSV19, PI3-E12, PI3-C9, PI3-A3, PI3-B5, PI3-A10, PI3-A12, PIA174, anti-14VP1, anti-89VP1, MV33, EV42, Fd138-80, Fd79, lenvervimab, AB65, AB68, TI-57, anivovetmab, fiztasovimab, regavirumab, sevirumab, alvircept sudotox, elipovimab, lafuvitug, teropavimab, and zinlirvimab.

12. Conjugation product according to claim 8 or 9, **characterized in that** the antibacterial antibody is an antibody targeting at least one of Pseudomonas aeruginosa and Staphylococcus aureus.

13. Conjugation product according to claim 8 or 9, **characterized in that** the anti-allergen antibody is an antibody targeting at least one of house dust mite, peanut, cat hairs, and pollen, and/or is chosen from the group consisting of omalizumab, mepolizumab, reslizumab, benralizumab, dupilumab, house dust mite Fab, house dust mite IgE, peanut-specific IgG, peanut-Fab, cat-Fel d 1, cat-Fel d 2, Bet v 1-IgE, and Phl p 5-Fab.

14. Conjugation product according to claim 8 or 9, **characterized in that** the anti-sperm cell antibody is chosen from the group consisting of anti-sperm IgA, anti-sperm IgG, and anti-sperm IgM.

15. Method for enhancing an inherent virus-binding efficacy, bacterium-binding efficacy, allergen-binding efficacy, or sperm-cell binding efficacy of an antigen binding moiety by covalently bonding it to a mucin binding moiety to result in a conjugation product according to any of the preceding claims.
